# EUROPEAN PATENT SPECIFICATION

(11) **EP 1 576 374 B1**
(45) Date of publication and mention of the grant of the patent: **06.08.2008**
(21) Application number: 03772547.0
(22) Date of filing: 27.10.2003
(51) Int. Cl.: G01N 33/68, C12Q 1/48, G01N 33/543

(54) **ENZYME ARRAY AND ASSAY**
ENZYMARRAY UND ENZYMTEST
MATRICE ET DOSAGE ENZYMATIQUES

(30) Priority: 25.10.2002 GB 0224872; 20.12.2002 WO PCT/EP02/14859; 23.05.2003 GB 0311946
(43) Date of publication of application: 21.09.2005
(73) Proprietor: SENSE PROTEOMIC LIMITED, Maidenhead, Berkshire SL6 7RJ (GB)
(72) Inventor: KOOPMANN, Jens-Oliver, Sense Proteomic Limited, Maidenhead, Berkhire, SL6 7RJ (GB); BLACKBURN, Jonathan M., Sense Proteomic Limited, Maidenhead, Berkshire, SL6 7RJ (GB)
(74) Representative: Peter, Beate
(86) International application number: PCT/IB2003/005427
(87) International publication number: WO 2004/038040

(56) References cited:
- WO-A-00/04382
- NELSON R W: "The use of bioreactive probes in protein characterization." MASS SPECTROMETRY REVIEWS. 1997 NOV-DEC, vol. 16, no. 6, November 1997 (1997-11), pages 353-376, XP002284011 ISSN: 0277-7037 -& DOGRUEL D ET AL: "RAPID TRYPTIC MAPPING USING ENZYMATICALLY ACTIVE MASS SPECTROMETER PROBE TIPS" ANALYTICAL CHEMISTRY, AMERICAN CHEMICAL SOCIETY. COLUMBUS, US, vol. 67, no. 23, 1 December 1995 (1995-12-01), pages 4343-4348, XP000540116 ISSN: 0003-2700
- ZHU H ET AL: "ANALYSIS OF YEAST PROTEIN KINASES USING PROTEIN CHIPS" NATURE GENETICS, NATURE AMERICA, NEW YORK, US, vol. 26, November 2000 (2000-11), pages 283-289, XP000994841 ISSN: 1061-4036
- NEWTON RUSSELL P ET AL: "Assay of adenosine 3',5'-cyclic monophosphate-dependent protein kinase activity by quantitative fast atom bombardment mass spectrometry" ANALYTICAL BIOCHEMISTRY, vol. 224, no. 1, 1995, pages 32-38, XP002285382 ISSN: 0003-2697

## Description

### CROSS REFERENCE TO RELATED APPLICATIONS

This application claims priority from GB0311946.8, filed May 23, 2003, GB0224872.2, filed October 25, 2002 and PCT/EP02/14859, filed December 20.

### FIELD OF THE INVENTION

The present invention relates to an enzyme assay and more particularly to a kinase assay for use with a laser desorption/ionisation mass spectrometer, such as a MALDI mass spectrometer.

### BACKGROUND TO THE INVENTION

Proteomic applications for mass spectrometry have seen a strong growth in recent years. Analytical methods used in proteomics are mainly based on 2D-gel electrophoresis for protein separation, and either mass spectrometry or Edman degradation for protein identification. The limitations of 2D gel electrophoresis include relatively poor resolution, sensitivity and reproducibility. As a result proteomic methods which avoid 2D-gel electrophoresis such as Isotope Coded Affinity Tag (ICAT)¹, Tandem Affinity Protein (TAP)² purification and the use of protein microarrays³ are gaining popularity.

Furthermore, these new methods have broadened the scope of proteomics from collecting and cataloguing differential expression data to a stage where relations between molecules can be assigned and this has been referred to as functional proteomics. Protein micro arrays have recently been used to analyze 119 yeast kinases ⁴and a major fraction of the yeast proteome ⁵.

Protein microarrays have been analyzed by enhanced chemi-luminescence (ECL), fluorescent or radioactive labels or via antibody based detection systems, but not to date by mass spectrometry.

The current reliance on the use of labeled ligands, such as antibodies or labeled probes, to analyze protein microarrays imposes constraints on the applications for protein microarrays. Hence a sensitive label free detection system would be of great advantage and would broaden the range of application to areas where labeled compounds either are not available, or are too expensive or where labeling would fundamentally alter the properties of the ligand, Such a label free method would be particularly useful in the early stages of drug discovery, where great numbers of compounds are screened against proteins.

Such a mass spectrometry probe, upon which an enzyme microarray has been fabricated, enables interrogation of enzymatic reactions and the effect compounds have thereon in a label-free manner by desorption and ionisation of reactants and products. The probe and methods are particularly useful in the drug discovery process, for example in hit series evaluation, lead optimization, predictive toxicogenomics and metabolite profiling.

The probes and method could however be used as a diagnostic tool to both diagnose disease states and monitor disease progression.

Nelson (1997 Nov-Dec) Mass Spectrometry Reviews, vol 16(6) pages 353-376 describes the use of bioreactive probes in protein characterization.

Zhu et al (Nov 2000), Nature Genetics, vol 26, pages 283-289 describes the analysis of yeast protein kinases using protein chips.

WO 00/04382 discloses protein arrays for the parallel, in vitro screening ofbiomolecular activity.

### SUMMARY OF THE INVENTION

According to the invention there is provided a method of determining the activity of an enzyme, or the effect a test compound has on the activity of the enzyme, using matrix-assisted laser desorption-ionisation mass spectrometry or desorption-ionisation on silicon mass spectrometry comprising:
i) providing a probe carrying an immobilized enzyme;
ii) optionally introducing the test compound;
iii) introducing one or more reactants to the immobilized enzyme for a time, and in a form sufficient for a reaction to take place;
iv) optionally drying the probe;
v) subjecting the probe to matrix-assisted laser desorption-ionisation mass spectometry or desorption-ionisation on silicon mass spectrometry; and
vi) determining the activity of an enzyme, or the effect the test compound had on the activity of the enzyme, by detecting the presence and/or absence of one or more products and/or the one or more reactants, **characterized in that** a layer resistant to non-specific protein binding is provided on the probe surface, and wherein the mass spectrometer is a laser desorption ionisation mass

Preferably the mass spectrometry uses a MALDI mass spectrometer. However, the skilled person will appreciate that numerous other mass spectrometry methods which enable the ionization event can also be used, including laser desorption-ionisation mass spectrometry, matrix-assisted laser desportion-ionisation (MALDI) mass spectrometry; and desorption-ionisation on silicon (DIOS) mass spectrometry. Clearly fourier transform mass spectrometry (FT/MS) methods such as fourier transform ion cyclotron resonance (FT-ICR) mass spectrometry can be used to enhance the mass accuracy of the various ionisation methods.

Whilst the method can be used to study any enzymatic reaction where one or more reactants are converted to one or more products and either the reactants and or the products can be discerned using e.g. MALDI mass spectrometry it is particularly suitable as a method for investigating kinases as all kinases use and / or generate a nucleotide tri phosphate (NTP) or a nucleotide di phosphate (NDP) e.g. adenine tri phosphate (ATP) or adenine di phosphate (ADP) which can be readily and distinguishably detected. The skilled person will understand that other nucleotide species such as guanine, uridine and cytosine may also be used, although adenine is preferred due to the fact it provides for a more sensitive assay as detection can be achieved at pico (10⁻¹²) Molar levels. Sensitivity is improved using MALDI mass spectrometry due to the enhancing effects of matrix.

In one embodiment step ii) is essential, and the effect the test compound has on the enzymatic activity is determined by comparison with the results obtained where the test compound is absent. The assay may be run on a single array, by running two or more assays in parallel, or by comparison to a standard. The test compound may be added pre, post or most preferably with the one or more reactants.

The method may be used to provide either a qualitive or quantitive result.

Preferably the method will determine the activity of one or more kinases or the effect a test compound has on the activity of one or more kinases by using MALDI mass spectrometry. Thus a kinase array for use in the method may comprise one or more kinases, for example, at least 10, through 25, 50, 75, 100, 200, 300 or more of the some 500 plus kinases of the kineome. These may be arranged on a microarray, each kinase being deposited at a discrete target area.

For a kinase assay the one or more reactants may comprise a phosphate donor, a phosphate acceptor and a divalent cation. The phosphate donor may be a phosphorylated substrate and the phosphate acceptor may be a nucleotide di phosphate (NDP). The simplicity of the method resides in the fact that these reactants are common to all kinase reactions thus enabling a single set of conditions to be applied across a range of kinases. This means the assay is robust and enables it be used for high throughput screening.

Of course it is possible to use discrete as well as generic substrates and examples of kinases and their substrates are shown in Tables 1 and 2, annexed hereto.

In one embodiment the phosphate donor is a nucleotide tri phosphate (NTP) and the phosphate acceptor is a substrate to be phosphorylated. Preferably the divalent cation (M²⁺) is magnesium or manganese.

In another embodiment the product is a nucleotide tri phosphate or a nucleotide di phosphate, the presence of which is detected. Of course since a typical kinase reaction is reversible the reactants may be the products and vice versa.

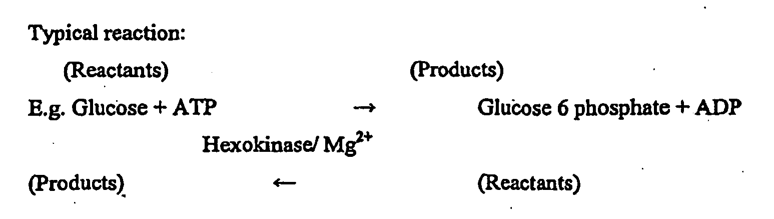

Typically the nucleotide tri phosphate or nucleotide di phosphate are detected as [NTP]⁻ or [NDP]⁻ and / or as one or more adduct peaks thereof. The one or more adduct peaks are typically adduct peaks with a monovalent cat ion (M⁺) (e.g. Na, K, Li.) depending on the reagents/buffers used. The one or more adduct peaks may, for example, include [ATP+M]⁻, [ATP+2M]⁻, and [ATP+3M]⁻ and/or [ADP+M]⁻, [ADP+2M]⁻, and [ADP+3M]⁻,

An important factor in being able to achieve good detection is in the selection of a low salt buffer. Preferably the low salt buffer is a "semi volatile buffer" such as, ammonium bicarbonate buffer. Such buffers do not leave a residue on evaporation as they are converted into gases, which in the case of ammonium bicarbonate are ammonia, carbon dioxide and water. Alternatively, since the reaction mix need only be a "low salt" buffer at the point of vaporisation/ionisation it would be possible to use a buffer containing a higher concentration of a semi-volatile salt and then, after the reaction is finished, remove the semi-volatile buffer in vacuo (either in the mass spec vacuum chamber or in an external vacuum chamber). This however is more complex and less desirable.

A further and significant feature of the invention resides in the fact that in a kinase assay the detected products/ reactants are small; typically less than 1000 daltons and consequently the mass spectrometry analysis can be conducted without having to overlay the probe with energy absorbing molecules (matrix). This simplifies and speeds up the procedure as well as saving costs. However, the addition of matrix increases sensitivity.

Where energy absorbing molecules are applied these should be applied to the probe in register with the immobilised enzyme.

The one or more reactants, and if present the test compound, are preferably introduced to the immobilised enzyme in a compartmentalised form, such as in the form of a droplet. Preferably the droplet has a volume of less than 1 microlitre.

Additionally it is preferred that the assay is conducted in a humid environment.

Of course, as well as kinases the method of the invention is applicable to other enzymes. Thus, it is possible to study enzyme reactions of immobilized proteins on protein arrays by mass spectrometry wherever the reactants and / or products are ionisable and where the enzymatic reaction leads to a mass change in the reactant and or product. This can be the case for Oxidoreductases, Transferases, Hydrolases, Lyases and Ligases.

Typical subclasses of enzymes from these groups of enzymes are listed in table 3 below:

**Table 3**

| Subclass | Name |
|---|---|
| | **EC1 Oxidoreductases** |
| **EC 1.1** | Acting on the CH-OH group of donors |
| **EC 1.2** | Acting on the aldehyde or oxo group of donors |
| **EC 1.3** | Acting on the CH-CH group of donors |
| **EC 1.4** | Acting on the CH-NH₂ group of donors |
| **EC 1.5** | Acting on the CH-NH group of donors |
| **EC 1.6** | Acting on NADH or NADPH |
| **EC 1.7** | Acting on other nitrogenous compounds as donors |
| **EC 1.8** | Acting on a sulfur group of donors |
| **EC 1.9** | Acting on a heme group of donors |
| **EC 1.10** | Acting on diphenols and related substances as donors |
| **EC 1.11** | Acting on a peroxide as acceptor |
| **EC 1.12** | Acting on hydrogen as donor |
| **EC 1.13** | Acting on single donors with incorporation of molecular oxygen (oxygenases) |
| **EC 1.14** | Acting on paired donors, with incorporation or reduction of molecular oxygen |
| **EC 1.15** | Acting on superoxide radicals as acceptor |
| **EC 1.16** | Oxidising metal ions |
| **EC 1.17** | Acting on CH₂ groups |
| **EC 1.18** | Acting on iron-sulfur proteins as donors |
| **EC 1.19** | Acting on reduced flavodoxin as donor |
| **EC 1.20** | Acting on phosphorus or arsenic in donors |
| **EC 1.21** | Acting on X-H and Y-H to form an X-Y bond |
| **EC 1.97** | Other oxidoreductases |
| **EC2** | **Transferases** |
| **EC 2.1** | Transferring one-carbon groups |
| **EC 2.2** | Transferring aldehyde or ketonic groups |
| **EC 2.3** | Acyltransferases |
| **EC 2.4** | Glycosyltransferases |
| **EC 2.5** | Transferring alkyl or aryl groups, other than methyl groups |
| **EC 2.6** | Transferring nitrogenous groups |
| **EC 2.7** | Transferring phosphorus-containing groups |
| **EC 2.8** | Transferring sulfur-containing groups |
| **EC 2.9** | Transferring selenium-containing groups |
| **EC3** | **Hydrolases** |
| **EC 3.1** | Acting on ester bonds |
| **EC 3.2** | Glycosylases |
| **EC 3.3** | Acting on ether bonds |
| **EC 3.4** | Acting on peptide bonds (peptidases) |
| **EC 3.5** | Acting on carbon-nitrogen bonds, other than peptide bonds |
| **EC 3.6** | Acting on acid anhydrides |
| **EC 3.7** | Acting on carbon-carbon bonds |
| **EC 3.8** | Acting on halide bonds |
| **EC 3.9** | Acting on phosphorus-nitrogen bonds |
| **EC 3.10** | Acting on sulfur-nitrogen bonds |
| **EC 3.11** | Acting on carbon-phosphorus bonds |
| **EC 3.12** | Acting on sulfur-sulfur bonds |
| **EC 3.13** | Acting on carbon-sulfur bonds |
| **EC4** | **Lyases** |
| **EC 4.1** | Carbon-carbon lyases |
| **EC 4.2** | Carbon-oxygen lyases |
| **EC 4.3** | Carbon-nitrogen lyases |
| **EC 4.4** | Carbon-sulfur lyases |
| **EC 4.5** | Carbon-halide lyases |
| **EC 4.6** | Phosphorus-oxygen lyases |
| **EC 4.99** | Other lyases |
| **EC6** | **Ligases** |
| **EC 6.1** | Forming carbon-oxygen bonds |
| **EC 6.2** | Forming carbon-sulfur bonds |
| **EC 6.3** | Forming carbon-nitrogen bonds |
| **EC 6.4** | Forming carbon-carbon bonds |
| **EC 6.5** | Forming phosphoric ester bonds |

Alternatively the method can be used to monitor enzymatic reactions involving co-substrates (also reactants in the context of this application) including NAD, NADP, NADH, NADPH, ATP, GTP, UTP, CTP, UDP-glucose, UDP-glucosamine, UDP-galactose, pyridoxalphosphate, UDP-*N*-acetyl-D-glucosamine, GDP-D-mannose, dTDP-6-deoxy-L-mannose, GDP-6-deoxy-D-talose, UDP-*N*-acetylmuramate, *S*)-3-hydroxyacyl-CoA, *S-*adenosyl-L-methionine, acetyl-CoA, L-selenoseryl-tRNA^{sec}, (*S*)-3-hydroxy-3-methylglutaryl-CoA, 5,10-methylentetrahydrofolate, ascorbate, 2-oxoglutarate, glutathione, pyruvate and tetrahydropteridine.

This is particularly useful when the substrates or products are not ionisable or when the reaction does not cause a mass change, as is seen for Isomerases (for example phenylalanine racemase which is ATP-hydrolysing).

More specifically, the enzyme is drawn from one or more of the group or groups of those enzyme families that are common drug targets, such as protein kinases (including serine/threonine kinases and tyrosine kinases), proteases (including serine proteases, cysteine proteases, aspartyl proteases and metalloproteinases), carboxylases, esterases, phosphodiesterases, protein phosphatases (including tyrosine phosphatases), G-protein coupled receptors, ATP-dependent chaperones, cyolooxygenases, cytochrome P450s, sialidases, and short-chain dehydrogenases/reductases.

A probe for use with a mass spectrometer can be provided, comprising a support having an electroconductive target surface thereon characterised in that the target surface comprises an array having a plurality of enzymes immobilised thereon.

The enzymes can be selected from the groups of enzymes listed above. Particularly preferred are those enzyme families that are common drug targets, particularly though not exclusively kinases.

Preferably the array is a micro array.

The enzymes are preferably attached to the probe as fusion proteins, typically via a tag, such as, for example, biotin, or a sh ble protein.

Related aspects to the invention are described in full in a number of the applicant's earlier patent applications including WO 01/57198 and are thus not dealt with in depth herein, but as others are as yet unpublished, such as GB 0224872.2, further supporting and related details are given below:

Thus, the probes referred to herein include microarrays, as well as arrays in which the protein spots will be visible to the naked eye, and are adapted so that they may be interrogated by means of laser desorption/ ionisation mass spectrometry, particularly, though not exclusively, matrix assisted laser desorption/ ionisation (MALDI).

Additional aspects include methods leading to the production of such a probe which can be interrogated by means of laser desorption/ ionisation mass spectrometry, particularly matrix assisted laser desorption/ionisation (MALDI) and methods of analysing such a probe or protein microarray.

Thus, the development of a MALDI MS-compatible protein microarray which term includes enzyme microarrays was complex since existing methods of forming protein microarrays did not transfer readily to a MALDI target. There are a number of reasons why this was the case, including the specialised nature of the probe surfaces and the potential for salts present in reaction buffers to interfere with the detection method.

Procedures known in the art for MALDI typically require the co-crystallization of the aqueous analyte with acidic energy absorbing molecules, or 'matrix', to promote ionization of the analytes (Karas and Hillenkamp, 1988). The method of co-crystallizing analyte and matrix for MALDI, as known in the art, typically results in a heterogeneous crystallization process and yields discrete, spatially separated crystals that each contains differing amounts of matrix and analyte. As a consequence it is often observed that individual crystals contain insufficient analyte for analysis by MALDI. This in turn results in a requirement for the analyser to sample multiple (i.e. 10-100 or more) discrete locations within a given target area in order to obtain a good analyte signal; this is sometime referred to as "the search for the sweet spot". This has previously prevented miniaturization since protein spots needed to be large. They were generally in the order of at least 0.5mm².

In order to generate MALDI MS-compatible protein microarrays, solutions for the aforementioned shortcomings of the prior art were required that enabled both miniaturization of the target areas and functional analysis of the arrayed proteins.

As defined herein a probe is a support which is capable of acting as a target in analysis by laser desorption/ionisation mass spectrometry, for example matrix assisted laser desorption/ionisation (MALDI). The probe carries the enzymes, e.g. kinases and the reactants (and optionally test compounds) are added. After a time sufficient for a reaction to take place, and products to be formed, the probes are dried and subjected to mass spectrometry. The reactants and or the products interact with the repeller lens of the ion-optic assembly found in laser desorption/ionisation time-of-flight (TOF) mass spectrometers of the art, such that the converted to gaseous ions which permits analysis. For example, the probes of the invention may be derived from targets for MALDI analysis as known in the art, which are treated such that a high affinity protein binding moiety e.g. streptavidin, avidin or neutravidin molecules are present on the probe surface which bind biotinylated enzymes for subsequent analysis. For example, conventional glass or gold MALDI targets may be used.

As defined herein a micro array is an array where the size of the discrete target areas i.e. the individual areas probed by a laser, is in the order of micrometers or less. Whilst at the upper end of the scale, around 1000 micrometers diameter, they may be visible to the naked eye at the lower end of the scale the discrete target areas will not be clearly distinguished by the naked eye.

The arrays will typically be arranged in matrices comprising several rows and columns. The number of discrete target areas will depend upon what is being screened though it is generally desirable to have a high density of these discrete areas on the probe surface as this will facilitate high through put screening. Typically a probe will comprise at least 10, more preferably at least 100, more preferably at least 1000 and as many as 10,000 or more target areas produced thereon. (Typically a probe surface will have an area of around 10,000mm² - a Bruker probe has an area of 10,292mm² although there is no requirement to use the whole of the probe and the microarray can be applied in one or more matrices thereon.) The actual density in a given matrices will depend upon the size of the discrete target area (which will typically be printed as a spot) and the spacing between adjacent spots. Thus the discrete target areas will typically be present at a density of greater than 1 discrete target areas per mm² within any matrices.

The enzyme is the moiety about which the reaction occurs.

The term "enzyme" as used herein is used to include both whole enzymes and sub units or domains thereof.

"Fusion protein" as used herein is used to refer to an enzyme, which has a tag, for example, a biotinylation consensus sequence or phleomycin/zeocin resistance binding protein attached thereto.

"Linker molecules" are molecules which function as their name suggests. They are molecules comprising functional groups which allow bridges to be formed between different molecules.

Another significant development enabling the "miniaturization" of an enzyme array formed on a MALDI target derives from the application of the Applicant's COVET technology described in WO 01/57198. Briefly, using this technology they are able to create from cDNA libraries expressed enzymes, which carry a "sequence tag" that can be used to capture the enzymes with a high affinity and in a specific orientation on the microarray surface. This firstly enables enzymes e.g. a kinase library to be stably immobilized such that leaching of enzymes from the surface is avoided and secondly the oriented immobilization of the fusion protein onto the surface ensure maximum biological activity.

Yet another significant aspect, when compared to current protein microarrays, is the provision of such a probe with an electro conductive surface. This surface which includes semi conductive surfaces is essential where the probe is to be subjected to MALDI MS analysis. Whilst the support could be made wholly of an electro conductive material (which term is used herein to include semi conducive materials) it is preferred to coat a rigid support, e.g. a glass, with an electro conductive material such as, for example, gold although any suitable metal, for example, silver, platinum, iridium, wolfram, copper, cobalt, nickel, and iron or mixtures thereof, or a semiconductor e.g. silicon oxide, graphite or germanium oxide could be used.

Where the probe or enzyme microarray is produced on e.g. a standard size microscope glass slide it can be mounted in an adapter, which carries it into a mass spectrometer. Such an adaptor is described in Applicant's co pending UK application no. 0216387.1.

A further significant feature of the present invention is the way the Applicant has overcome the problems caused by non specific protein binding. The Applicant has overcome this particular problem by providing a layer resistant to non specific protein binding onto the probe surface. More particularly, the microarray surface is modified by the inclusion of a layer of molecules which repel proteins. These protein repellant molecules which include, for example, polyethylene glycol may be bound to the probe surface via a linker, such as, for example, a poly amino acid which readily binds to e.g. a glass or gold surface and whose amino or carboxyl side groups can be used to bind the protein repellant molecules such that they reach out from the probe surface. The skilled man will appreciate that other functionalized molecules could be used. Preferably the enzyme binding moieties are incorporated in a position where they extend out from the surface. Preferred enzyme binding moieties include e.g. biotin, biotin-neutravidin, and bleomycin, and these and other moieties can be incorporated into the layer either via these functional groups on the linker molecules and/ or via functional groups on the protein repellant molecules. Typically the affinity capture moieties are incorporated in small proportions (typically less than 20%) relative to the protein repellant molecules.

In this way the Applicant has been able to introduce the enzyme capture moieties not only in a homogeneous, spatial defined arrangement but also in a manner which enables high affinity binding in a specific manner. The resulting surface combines selectivity for the capture of biological macromolecules on the probe with reduced non specific binding of the type commonly observed on underivatised glass or metal surfaces and additionally results in a homogeneous distribution and orientation of the captured biological macromolecules.

The component molecules responsible for repelling non specific proteins include molecules which are generally hydrophilic in nature. They include polymers, such as, for example, polyethylene glycol, dextran, polyurethane and polyacrylamide and self assembled monolayers (SAM). Preferably the polymers comprise one or more functional side groups via which the protein capturing moieties can be attached. In the case of polyethylene glycol the functional group is a hydroxyl group. The molecules responsible for repelling non specific proteins may be bound directly to the surface as in, for example the case of SAM's or they may be attached via a linker. Particularly preferred as linkers are poly amino acids such as, for example, poly L lysine, poly L aspartic acid, poly L glutamic acid or mixtures thereof.

These have amino or carboxy side chains via which the molecules responsible for repelling non specific proteins can be attached and which can additionally be used to attach the protein capturing moieties. Alternatively, or in addition, the protein capturing moieties can be attached via the component molecules responsible for repelling non specific proteins.

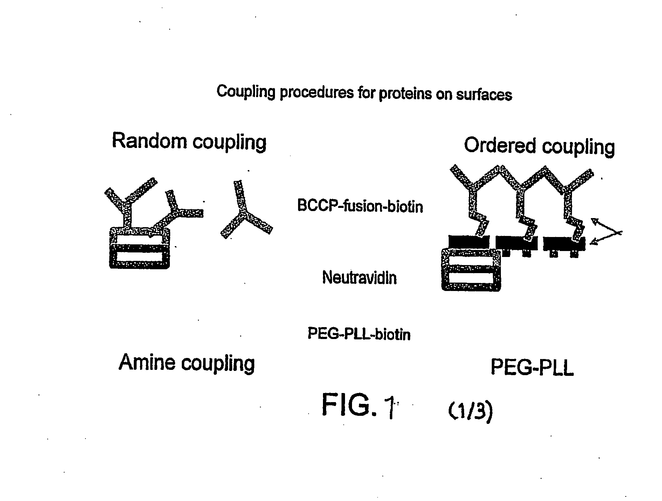

Fig 1 illustrates the binding of such molecules and contrasts the defined orientation which can be achieved by this ordered coupling compared to that achieved using current antibody binding techniques which result in random coupling.

In a preferred embodiment the probe has as it's enzyme capture moieties either a biotin binder e.g. neutravidin, avidin or streptavidin or a bleomycin resistant protein binder e.g. bleomycin. The enzymes are bound to the probe to create a protein microarray by printing a plurality of bacterial, yeast, sf9 or mammalian cell lysates containing fusion proteins in which a high affinity tag e.g. biotin or zeocin resistant protein (ZRP) is expressed onto the capture surface. Proteins are derived from the expression of a cDNA library and each individual clone is tagged at the C-terminus and/ or on the N-terminus with a consensus sequence, which will enable high affinity recognition of the enzyme even in the presence of the otherwise protein repellent molecules. Only the recombinant, tagged enzyme can recognise the capture surface and other proteins from the lysate can be washed away as they do not bind to the protein repellent surface and do not have a high affinity to the protein binding moieties present in the layer.

A method of producing an enzyme microarray for use with a mass spectrometer comprising providing a probe and depositing the enzyme in registration with the protein capturing moieties in the discrete target area can be provided.

Yet a further aspect provides a method of analyzing a probe in which energy absorbing molecules are deposited in a manner which denatures and thus unbinds an enzyme from a protein capturing moiety leaving the denatured enzyme lying unbound on the surface.

The energy absorbing molecules form a homogenous layer of crystals in a discrete location in registration with the protein capturing moieties and captured enzyme.

The homogenous layer of crystals is substantially continuous such that individual crystals are not visible at a 100 fold magnification and there are no visible gaps. It also has a substantially uniform depth, such that there is no apparent variation in crystal size at a 100 fold magnification.

The energy absorbing molecules are deposited onto the surface in a non aqueous solvent and the non aqueous solvent is evaporated off. Preferably the non aqueous solvent is an organic solvent, such as, for example, acetone or butanone.
Preferably the non aqueous solvent includes a modifier which controls the rate of evaporation such that crystallization of the energy absorbing molecules occurs on the probe. Suitable modifiers include glycerol, polyethylene glycol and thioglycerol.
Preferably the energy absorbing molecules are deposited in a mixture of from 80 -99.9%, preferably 99% organic solvent e.g. acetone to 20 - 0.1%, preferably 1% of modifier e.g. glycerol (vol/vol) .Typical energy absorbing molecules include crystals of α-cyano-4-hydroxy-cinnamic acid, sinapinic acid, gentisic acid, nifidine, succinic acid, 1,8,9,-anthracenitriol, 3-Indoleacrylic acid, 2-(hydroxyphenylazo) benzoe-acid, 4-nitroanilin and combinations thereof.

Preferably the energy absorbing molecules are deposited in registration with the protein and each protein spot is overlaid with a similar sized spot of the energy absorbing molecules.

In order to achieve a high density of individual samples on the microarray the energy absorbing molecules need to be arranged in microcrystals on the surface. The matrix forms a homogenous layer of flat crystals without significant gaps between them and can be deposited in very small quantities on the microarray.

In contrast to the prior art in which matrix and analyte are co crystallized in an aqueous solvent, the Applicant uses two distinct steps in which first the protein is deposited in an aqueous solvent and then the energy absorbing molecules are deposited such that they crystallise out from the non aqueous solvent on the probe. This has the advantage that the protein is deposited in its biological form. However, using a non aqueous solvent to deliver the energy absorbing molecules allows the formation of a homogenous layer of microcrystals.

This has two benefits. First the formation of a homogenous layer means it is not necessary to search for a sweet spot as the homogenous layer guarantees protein in the presence of energy absorbing molecules and secondly it results in more accurate measurement due to the even nature of the layer.

### BRIEF DESCRIPTION OF THE DRAWING

The various aspects of the invention will now be described, by way of example only, with reference to the following figures and examples in which:
Fig 1 shows the orientated binding of the enzymes to a probe
Fig 2, shows the detection of ADP and ATP using mass spectrometry, and
Fig 3 also shows creatine phosphate mediated ATP synthesis on a surface

### DETAILED DESCRIPTION

### EXAMPLE 1

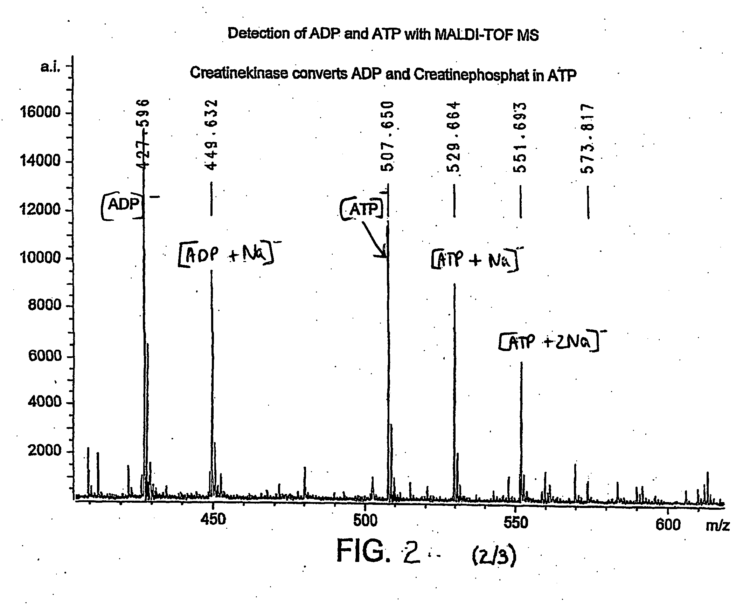

Referring to Fig 2, ATP was enzymatically synthesized from the reaction of ADP, creatine phosphate and creatine kinase (also known as creatine phosphokinase) in 25 mM ammonium bicarbonate at pH 7.4. [ADP]⁻ was detected at 427.6 dalton and [ADP+Na]⁻ at 449.6 dalton. The products of the creatine phosphate kinase reaction were detected at 507.6, 529.6, 551.6 and 573.8, which fit well with the expected molecular weight of [ATP]⁻ and three ATP sodium adducts [ATP+Na]⁻, [ATP+2Na]⁻ and [ATP+3Na]⁻ .

Control reactions in which either one of the substrates ADP or creatine phosphate or the enzyme creatine phosphate kinase was omitted didn't show ATP peaks.

### EXAMPLE 2

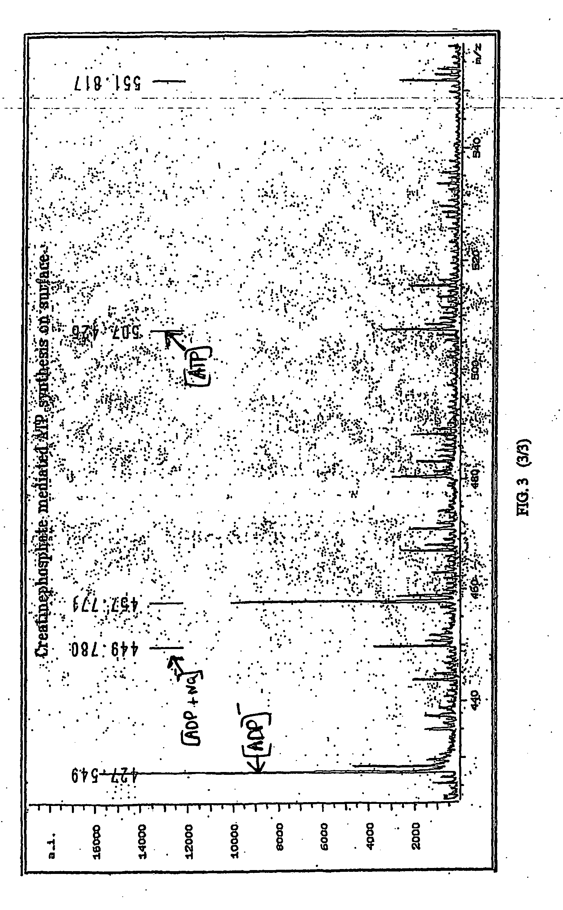

Referring to Fig 3 the example demonstrates the biotinylation, capture, and desalting of creatine kinase from rabbit muscle on a PEG-PLL-Biotin Neutravidin coated probe for analysis by MALDI mass spectrometry and the enzymatic activity of the immobilized kinase using MALDI mass spectrometry.

### Material:

Creatine kinase from rabbit muscle ATP: creatine N-phosphotransferase ADP, creatine phosphate, 1 mM Tris HCl pH 7.5, 1 mM MgCl₂, gold coated glass slide, PEG-PLL-biotin, Neutravidin

### Solutions:

Washing buffer: 1 mM Tris-HCl pH 7.5 with 0.1% Triton X-100; desalting buffer: 1 mM Tris-HCl pH 7.5.

### Affinity capture polymer synthesis

The poly-L-lysine PEG-biotin (PEG-PLL-biotin) was synthesized according to the protocol of Ruiz Taylor ⁶. Briefly, 100 mg poly-L-lysine (average size 17-30 kDa; Sigma, Dorset, UK) was reacted with 109 mg mPEG-SPA and 1.1 mg biotinPEG-CO-NHS (Shearwater Corporation, Huntsville, Alabama) in 1ml 100 mM sodium carbonate buffer pH 9 for a period of 30 minutes. The reaction was terminated by dialysis in 1 mM Tris-HCl pH 7.5 overnight. The product from this reaction was called PEG-PLL-biotin (1% PEG derivatives contain a biotin headgroup).

### Biotinylation of Creatine kinase

Creatine kinase (100 mg) was dissolved in 1 ml 1 mM Tris HCl pH 7.5 and 1mg of EZ link biotin PEO amine and 1 mg ethylene diamine carbodiimide were reacted for 20 minutes at room temperature.

### Affinity capture surface preparation

Protein micro array probes were thoroughly cleaned before use with sequential washing steps in acetone, acetonitrile, double distilled water and dried under nitrogen. Freshly prepared affinity capture polymer PEG-PLL-Biotin was then pipetted on the surface of the probe and was then evenly distributed on the surface by covering it with Nesco film (Azwell Inc., Osaka, Japan). After 30 min the probe was washed in 1 mM Tris-HCl pH 7.5, dried under nitrogen and then coated with 0.5 mg/ml neutravidin for one hour at RT in a humid chamber. The probe was then rinsed with washing buffer, washed twice with desalting buffer and dried under nitrogen. The surface was now ready to be used as a highly specific affinity capture device for biotinylated macromolecules.

### Capture and detection of biotinylated proteins on the probe surface

A PLL-PEG-biotin neutravidin surface on a MALDI target was overlaid with 50 ng of biotinylated creatine kinase (Roche, Mannheim, Ger) The biotinylated protein was captured for a period of 2 hours on the MALDI target in a humid chamber to prevent drying, washed twice with washing buffer followed by two washes with desalting buffer, surface was dried under nitrogen and overlaid with 300 nl of a saturated solution of cyano-4-hydroxycinnamic acid in acetone.

### Monitoring the kinase activity of the immobilized Creatine Kinase on the protein array

The array with the immobilized creatine kinase is washed with 100 ml 1 mM Tris-HCl pH 7.5 and is then overlaid with a mixture 1 mM creatine phosphate, 1 mM ADP, 1 mM MgCl2 and 25 mM ammoniumbicarbonate buffer in a volume less than 1 microlitre. The enzyme and the substrates are incubated in a humid chamber at 37°C for a period of 30 minutes. Reactions which omitted either ADP, creatine phosphate or the kinase were run in parallel as specificity controls.

### Results

Fig 3 shows the detection of ADP and ATP. ATP was enzymatically synthesized from the reaction of ADP, creatine phosphate and creatine kinase in 25 mM ammonium bicarbonate at pH 7.4. [ADP]⁻ was detected at 427.6 dalton and [ADP+Na]⁻ 449.6 dalton. The products of the creatine kinase reaction were detected at 507.6, 529.6, 551.6 and 573.8, which fits well with the expected molecular weight of [ATP]⁻ and three ATP sodium adducts [ATP+Na]⁻, [ATP+2Na]⁻ and [ATP+3Na]⁻.

Control reactions in which either one of the substrates ADP or creatine phosphate or the enzyme creatine kinase was omitted didn't show ATP peaks.

1 Gygi, S. P., Rist, B., Gerber, S. A., Turecek, F. Gelb, M. H. and Aebersold, R..Nat Biotechnol.1999;17,994-999

2 Gavin AC, Bosche M, Krause R, Grandi P, Marzioch M, Bauer A, Schultz J, Rick JM, Michon AM, Cruciat CM, Remor M, Hofert C, Schelder M, Brajenovic M, RuffnerH, Merino. A, Klein K, Hudak M, Dickson D, Rudi T, Gnau V, Bauch A, Bastuck S, Huhse B, Leutwein C, Heurtier MA, Copley RR, Edelmann A, Querfurth E, Rybin V, Drewes G, Raida M, Bouwmeester T, Bork P, Seraphin B, Kuster B, Neubauer G, Superti-Furga G.. Nature 2002; 415(6868),141-147.
3 MacBeath, G., and Schreiber, S. F. Science 2000; 289, 1760-1763
4 Zhu H, Klemic JF, Chang S, Bertone P, Casamayor A, Klemic KG, Smith D, Gerstein M, Reed MA, Snyder M. Nat Genet.2000; 26(3):283-9
5 Zhu H, Bilgin M, Bangham R, Hall D, Casamayor A, Bertone P, Lan N, Jansen R, Bidlingmaier S, Houfek T, Mitchell T, Miller P, Dean RA, Gerstein M, Snyder M. Science 2001; 293, 2101-2105
6 Natsume, T., Nakayama, H., Isobe, T. Trends Biotechnolo 2001; 10, 28-33

**Table 1.**

| **Swiss Prot Accession number** | **Protein name** |
|---|---|
| P43403 | TYROSINE-PROTEIN KINASE ZAP-70 (70 KDA ZETA-ASSOCIATED PROTEIN) (SYK-RELATED TYROSINE KINASE) |
| P07947 | PROTO-ONCOGENE TYROSINE-PROTEIN KINASE YES (P61-YES) (C-YES) |
| O60285 | PROBABLE SERINE/THREONINE-PROTEIN KINASE KIAA0537 |
| P30291 | WEE1-LIKE PROTEIN KINASE (WEE1HU) |
| P35916 | VASCULAR ENDOTHELIAL GROWTH FACTOR RECEPTOR 3 PRECURSOR (VEGFR-3) (TYROSINE-PROTEIN KINASE RECEPTOR FLT4) |
| P17948 | VASCULAR ENDOTHELIAL GROWTH FACTOR RECEPTOR 1 PRECURSOR (VEGFR-1) (TYROSINE-PROTEIN KINASE RECEPTOR FLT) (FLT-1) (TYROSINE-PROTEIN KINASE FRT) |
| O75385 | SERINE/THREONINE-PROTEIN KINASE ULK1 (UNC-51-LIKE KINASE 1) |
| P30530 | TYROSINE-PROTEIN KINASE RECEPTOR UFO PRECURSOR (AXL ONCOGENE) |
| Q06418 | TYROSINE-PROTEIN KINASE RECEPTOR TYRO3 PRECURSOR (TYROSINE-PROTEIN KINASE RSE) (TYROSINE-PROTEIN KINASE SKY) (TYROSINE-PROTEIN KINASE DTK) |
| P29597 | NON-RECEPTOR TYROSINE-PROTEIN KINASE TYK2 |
| 043914 | TYRO PROTEIN TYROSINE KINASE-BINDING PROTEIN PRECURSOR (DNAX-ACTIVATION PROTEIN 12) |
| P42681 | TYROSINE-PROTEIN KINASE TXK |
| P33981 | DUAL SPECIFICITY PROTEIN KINASE TTK (PYT) |
| P04629 | HIGH AFFINITY NERVE GROWTH FACTOR RECEPTOR PRECURSOR (TRK1 TRANSFORMING TYROSINE KINASE PROTEIN) (P140-TRKA) (TRK-A) |
| Q02763 | ANGIOPOIETIN 1 RECEPTOR PRECURSOR (TYROSINE-PROTEIN KINASE RECEPTOR TIE-2) (TYROSINE-PROTEIN KINASE RECEPTOR TEK) (P140 TEK) (TUNICA INTERNA ENDOTHELIAL CELL KINASE) |
| P35590 | TYROSINE-PROTEIN KINASE RECEPTOR TIE-1 PRECURSOR |
| P36897 | TGF-BETA RECEPTOR TYPE I PRECURSOR (TGFR-1) (TGF-BETA TYPE I RECEPTOR) (SERINE/THREONINE-PROTEIN KINASE RECEPTOR R4) (SKR4) (ACTIVIN RECEPTOR-LIKE KINASE 5) (ALK-5) |
| Q15569 | TESTIS-SPECIFIC PROTEIN KINASE 1 |
| P42680 | TYROSINE-PROTEIN KINASE TEC |
| O76039 | SERINE/THREONINE-PROTEIN KINASE 9 |
| Q13043 | SERINE/THREONINE PROTEIN KINASE 4 (STE20-LIKE KINASE MST1) (MST-1) (MAMMALIAN STE20-LIKE PROTEIN KINASE 1) (SERINE/THREONINE PROTEIN KINASE KRS-2) |
| Q13188 | SERINE/THREONINE PROTEIN KINASE 3 (STE20-LIKE KINASE MST2) (MST-2) (MAMMALIAN STE20-LIKE PROTEIN KINASE 2) (SERINE/THREONINE PROTEIN KINASE KRS-1) |
| P51957 | SERINE/THREONINE PROTEIN KINASE 2 (SERINE/THREONINE-PROTEIN KINASE NRK2) |
| O00506 | SERINE/THREONINE PROTEIN KINASE 25 (STERILE 20/OXIDANT STRESS-RESPONSE KINASE 1) (STE20/OXIDANT STRESS RESPONSE KINASE-1) (SOK-1) (STE20-LIKE KINASE) |
| Q9Y6E0 | SERINE/THREONINE PROTEIN KINASE 24 (STE20-LIKE KINASE MST3) (MST-3) (MAMMALIAN STE20-LIKE PROTEIN KINASE 3) |
| Q9UPE1 | SERINE/THREONINE PROTEIN KINASE 23 (MUSCLE-SPECIFIC SERINE KINASE 1) (MSSK-1) |
| O75716 | SERINE/THREONINE PROTEIN KINASE 16 (PROTEIN KINASE PKL12) (MYRISTOYLATED AND PALMITOYLATED SERINE-THREONINE KINASE) (MPSK) (TGF-BETA STIMULATED FACTOR 1) (TSF-1) (HPSK) |
| Q15831 | SERINE/THREONINE-PROTEIN KINASE 11 (SERINE/THREONINE-PROTEIN KINASE LKB1) |
| O94804 | SERINE/THREONINE-PROTEIN KINASE 10 (LYMPHOCYTE-ORIENTED KINASE) |
| P12931 | PROTO-ONCOGENE TYROSINE-PROTEIN KINASE SRC (P60-SRC) (C-SRC) |
| Q99611 | SELENIDE,WATER DIKINASE 2 (SELENOPHOSPHATE SYNTHETASE 2) (SELENIUM DONOR PROTEIN 2) |
| P49903 | SELENIDE, WATER DIKINASE 1 (SELENOPHOSPHATE SYNTHETASE 1) (SELENIUM DONOR PROTEIN 1) |
| Q9UEW8 | STE20/SPS1-RELATED PROLINE-ALANINE RICH PROTEIN KINASE (STE-20 RELATED KINASE) (DCHT) |
| Q9NYY3 | SERINE/THREONINE-PROTEIN KINASE SNK (SERUM INDUCIBLE KINASE) |
| P57059 | PROBABLE SERINE/THREONINE PROTEIN KINASE SNF1LK |
| Q9BPZ7 | STRESS-ACTIVATED MAP KINASE INTERACTING PROTEIN 1 (SAPK INTERACTING PROTEIN 1) (PUTATIVE RAS INHIBITOR JC310) |
| O00141 | SERINE/THREONINE-PROTEIN KINASE SGK (SERUM/GLUCOCORTICOID-REGULATED KINASE) |
| O94768 | SERINE/THREONINE KINASE 17B (DAP KINASE-RELATED APOPTOSIS-INDUCING PROTEIN KINASE 2) |
| Q9UEE5 | SERINE/THREONINE KINASE 17A (DAP KINASE-RELATED APOPTOSIS-INDUCING PROTEIN KINASE 1) |
| P34925 | TYROSINE-PROTEIN KINASE RYK PRECURSOR |
| Q01974 | TYROSINE-PROTEIN KINASE TRANSMEMBRANE RECEPTOR ROR2 PRECURSOR (NEUROTROPHIC TYROSINE KINASE, RECEPTOR-RELATED 2) |
| Q13516 | PROTEIN KINASE C-BINDING PROTEIN RACK17 (PROTEIN KINASE C BINDING PROTEIN 2) |
| Q13546 | SERINE/THREONINE PROTEIN KINASE RIP (CELL DEATH PROTEIN RIP) (RECEPTOR INTERACTING PROTEIN) |
| Q13308 | TYROSINE-PROTEIN KINASE-LIKE 7 PRECURSOR (COLON CARCINOMA KINASE-4) (CCK-4) |
| Q13882 | TYROSINE-PROTEIN KINASE 6 (BREAST TUMOR KINASE) (TYROSINE-PROTEIN KINASE BRK) |
| P78527 | DNA-DEPENDENT PROTEIN KINASE CATALYTIC SUBUNIT (DNA-PKCS) (DNPK1) |
| Q13523 | SERINElTHREONINE-PROTEIN KINASE PRP4 HOMOLOG |
| P53350 | SERINE/THREONINE-PROTEIN KINASE PLK (PLK-1) (SERINE-THREONINE PROTEIN KINASE 13) (STPK13) |
| P51817 | PROTEIN KINASE PKX1 |
| Q16513 | PROTEIN KINASE C-LIKE 2 (PROTEIN-KINASE C-RELATED KINASE 2) |
| Q16512 | PROTEIN KINASE C-LIKE 1 (PROTEIN-KINASE C-RELATED KINASE 1) (PROTEIN KINASE C-LIKE PKN) (SERINE-THREONINE PROTEIN KINASE N) |
| P00558 | PHOSPHOGLYCERATE KINASE 1 (PRIMER RECOGNITION PROTEIN 2) (PRP 2) |
| Q9P286 | SERINE/THREONINE-PROTEIN KINASE PAK 5 (P21-ACTIVATED KINASE 5) (PAK-5) |
| O75914 | SERINE/THREONINE-PROTEIN KINASE PAK 3 (P21 -ACTIVATED KINASE 3) (PAK-3)(BETA-PAK) |
| Q13177 | SERINE/THREONINE-PROTEIN KINASE PAK 2 (P21-ACTIVATED KINASE 2) (PAK-2) (PAK65) (GAMMA-PAK) (S6/H4 KINASE) |
| Q13153 | SERINE/THREONINE-PROTEIN KINASE PAK 1 (P21-ACTIVATED KINASE 1) (PAK-1) (P65-PAK) (ALPHA-PAK) |
| O43422 | 52 KDA REPRESSOR OF THE INHIBITOR OF THE PROTEIN KINASE (P581PK-INTERACTING PROTEIN) (58 KDA INTERFERON-INDUCED PROTEIN KINASE-INTERACTING PROTEIN) (P52RIPK) (DEATH ASSOCIATED PROTEIN 4) |
| Q99435 | PROTEIN KINASE C-BINDING PROTEIN NELL2 PRECURSOR (NEL-LIKE PROTEIN 2) (NEL-RELATED PROTEIN 2) |
| Q92832 | PROTEIN KINASE C-BINDING PROTEIN NELL1 PRECURSOR (NEL-LIKE PROTEIN 1) (NEL-RELATED PROTEIN 1) |
| P51956 | SERINE/THREONINE-PROTEIN KINASE NEK3 (NIMA-RELATED PROTEIN KINASE 3) (HSPK 36) |
| P51955 | SERINE/THREONINE-PROTEIN KINASE NEK2 (NIMA-RELATED PROTEIN KINASE 2) (NIMA-LIKE PROTEIN KINASE 1) (HSPK 21) |
| P15531 | NUCLEOSIDE DIPHOSPHATE KINASE A (NDK A) (NDP KINASE A) (TUMOR METASTATIC PROCESS-ASSOCIATED PROTEIN) (METASTASIS INHIBITION FACTOR NM23) (NM23-H1) |
| Q9ULX6 | NEIGHBOR OF A-KINASE ANCHORING PROTEIN 95 (HOMOLOGOUS TO AKAP95 PROTEIN) (HA95) (HELICASE A-BINDING PROTEIN 95) (HAP95) |
| Q13163 | DUAL SPECIFICITY MITOGEN-ACTIVATED PROTEIN KINASE KINASE 5 (MAP KINASE KINASE 5) (MAPKK 5) (MAPK/ERK KINASE 5) |
| P45985 | DUAL SPECIFICITY MITOGEN-ACTIVATED PROTEIN KINASE KINASE 4 (MAP KINASE KINASE 4) (JNK ACTIVATING KINASE 1) (C-JUN N-TERMINAL KINASE KINASE 1) (JNKK) (SAPK/ERK KINASE 1) (SEK1) |
| P46734 | DUAL SPECIFICITY MITOGEN-ACTIVATED PROTEIN KINASE KINASE 3 (MAP KINASE KINASE 3) (MAPKK 3) (MAPK/ERK KINASE 3) |
| P36507 | DUAL SPECIFICITY MITOGEN-ACTIVATED PROTEIN KINASE KINASE 2 (MAP KINASE KINASE 2) (MAPKK 2) (ERK ACTIVATOR KINASE 2) (MAPK/ERK KINASE 2) (MEK2) |
| Q02750 | DUAL SPECIFICITY MITOGEN-ACTIVATED PROTEIN KINASE KINASE 1 (MAP KINASE KINASE 1) (MAPKK 1) (ERKACTIVATOR KINASE 1) (MAPK/ERK KINASE 1)(MEK1) |
| P49137 | MAP KINASE-ACTIVATED PROTEIN KINASE 2 (MAPK-ACTIVATED PROTEIN KINASE 2) (MAPKAP KINASE 2) (MAPKAPK-2) |
| Q16539 | MITOGEN-ACTIVATED PROTEIN KINASE 14 (MITOGEN-ACTIVATED PROTEIN KINASE P38) (MAP KINASE P38) (CYTOKINE SUPPRESSIVE ANTI-INFLAMMATORY DRUG BINDING PROTEIN) (CSAID BINDING PROTEIN) (CSBP) (MAX-INTERACTING PROTEIN 2) (MAP KINASE MX12) |
| P53778 | MITOGEN-ACTIVATED PROTEIN KINASE 12 (EXTRACELLULAR SIGNAL-REGULATED KINASE 6) (ERK-6) (ERK5) (STRESS-ACTIVATED PROTEIN KINASE-3) (MITOGEN-ACTIVATED PROTEIN KINASE P38 GAMMA) (MAP KINASE P38 GAMMA) |
| Q15759 | MITOGEN-ACTIVATED PROTEIN KINASE 11 (MITOGEN-ACTIVATED PROTEIN KINASE P38 BETA) (MAP KINASE P38 BETA) (P38B) (P38-2) (STRESS-ACTIVATED PROTEIN KINASE-2) |
| P53779 | MITOGEN-ACTIVATED PROTEIN KINASE 10 (STRESS-ACTIVATED PROTEIN KINASE JNK3) (C-JUN N-TERMINAL KINASE 3) (MAP KINASE P49 3F12) |
| P45984 | MITOGEN-ACTIVATED PROTEIN KINASE 9 (STRESS-ACTIVATED PROTEIN KINASE JNK2) (C-JUN N-TERMINAL KINASE 2) (JNK-55) |
| P45983 | MITOGEN-ACTIVATED PROTEIN KINASE 8 (STRESS-ACTIVATED PROTEIN KINASE JNK1) (C-JUN N-TERMINAL KINASE 1) (JNK-46) |
| Q13164 | MITOGEN-ACTIVATED PROTEIN KINASE 7 (EXTRACELLULAR SIGNAL-REGULATED KINASE 5) (ERK-5) (ERK4) (BMK1 KINASE) |
| Q16659 | MITOGEN-ACTIVATED PROTEIN KINASE 6 (EXTRACELLULAR SIGNAL-REGULATED KINASE 3) (ERK-3) (MAP KINASE ISOFORM P97) (P97-MAPK) |
| P31152 | MITOGEN-ACTIVATED PROTEIN KINASE 4 (EXTRACELLULAR SIGNAL-REGULATED KINASE 4) (ERK-4) (MAP KINASE ISOFORM P63) (P63-MAPK) |
| P27361 | MITOGEN-ACTIVATED PROTEIN KINASE 3 (EXTRACELLULAR SIGNAL-REGULATED KINASE 1) (ERK-1) (INSULIN-STIMULATED MAP2 KINASE) (MAP KINASE 1) (MAPK 1) (P44-ERK1) (ERT2) (P44-MAPK) (MICROTUBULE-ASSOCIATED PROTEIN-2 KINASE) |
| P28482 | MITOGEN-ACTIVATED PROTEIN KINASE 1 (EXTRACELLULAR SIGNAL-REGULATED KINASE 2) (ERK-2) (MITOGEN-ACTIVATED PROTEIN KINASE 2) (MAP KINASE 2) (MAPK 2) (P42-MAPK) (ERT1) |
| Q12866 | PROTO-ONCOGENE TYROSINE-PROTEIN KINASE MER PRECURSOR (C-MER) (RECEPTOR TYROSINE KINASE MERTK) |
| P42679 | MEGAKARYOCYTE-ASSOCIATED TYROSINE-PROTEIN KINASE (TYROSINE-PROTEIN KINASE CTK) (PROTEIN KINASE HYL) (HEMATOPOIETIC CONSENSUS TYROSINE-LACKING KINASE) |
| P20794 | SERINE/THREONINE-PROTEIN KINASE MAK (MALE GERM CELL-ASSOCIATED KINASE) |
| P29966 | MYRISTOYLATED ALANINE-RICH C-KINASE SUBSTRATE (MARCKS) (PROTEIN KINASE C SUBSTRATE, 80 KDA PROTEIN, LIGHT CHAIN) (PKCSL) (80K-L PROTEIN) |
| Q99558 | MITOGEN-ACTIVATED PROTEIN KINASE KINASE KINASE 14 (NF-KAPPA BETA-INDUCING KINASE) (SERINE/THREONINE PROTEIN KINASE NIK) (HSNIK) |
| Q02779 | MITOGEN-ACTIVATED PROTEIN KINASE KINASE KINASE 10 (MIXED LINEAGE KINASE 2) (PROTEIN KINASE MST) |
| P80192 | MITOGEN-ACTIVATED PROTEIN KINASE KINASE KINASE 9 (MIXED LINEAGE KINASE 1) |
| P41279 | MITOGEN-ACTIVATED PROTEIN KINASE KINASE KINASE 9 (COT PROTO-ONCOGENE SERINE/THREONINE-PROTEIN KINASE) (C-COT) (CANCER OSAKA THYROID ONCOGENE) |
| O43318 | MITOGEN-ACTIVATED PROTEIN KINASE KINASE KINASE 7 (TRANSFORMING GROWTH FACTOR-BETA-ACTIVATED KINASE 1) (TGF-BETA-ACTIVATED KINASE 1) |
| 095382 | MITOGEN-ACTIVATED PROTEIN KINASE KINASE KINASE 6 |
| Q99683 | MITOGEN-ACTIVATED PROTEIN KINASE KINASE KINASE 5 (MAPK/ERK KINASE KINASE 5) (MEK KINASE 5) (MEKK 5) (APOPTOSIS SIGNAL-REGULATING KINASE 1) (ASK-1) |
| Q9Y6R4 | MITOGEN-ACTIVATED PROTEIN KINASE KINASE KINASE 4 (MAPK/ERK KINASE KINASE 4) (MEK KINASE 4) (MEKK 4) (MAP THREE KINASE 1) |
| Q99759 | MITOGEN-ACTIVATED PROTEIN KINASE KINASE KINASE 3 (MAPK/ERK KINASE KINASE 3) (MEK KINASE 3) (MEKK 3) |
| Q9Y2U5 | MITOGEN-ACTIVATED PROTEIN KINASE KINASE KINASE 2 (MAPK/ERK KINASE KINASE 2) (MEK KINASE 2) (MEKK 2) |
| Q13233 | MITOGEN-ACTIVATED PROTEIN KINASE KINASE KINASE 1 (MAPK/ERK KINASE KINASE 1) (MEK KINASE 1) (MEKK 1) |
| P07948 | TYROSINE-PROTEIN KINASE LYN |
| P06239 | PROTO-ONCOGENE TYROSINE-PROTEIN KINASE LCK (P56-LCK) (LSK) (T CELL-SPECIFIC PROTEIN-TYROSINE KINASE) |
| P43405 | TYROSINE-PROTEIN KINASE SYK (SPLEEN TYROSINE KINASE) |
| P08922 | PROTO-ONCOGENE TYROSINE-PROTEIN KINASE ROS PRECURSOR |
| P04049 | RAF PROTO-ONCOGENE SERINEITHREONINE-PROTEIN KINASE (RAF-1) (C-RAF) |
| P31749 | RAC-ALPHA SERINE/THREONINE KINASE (RAC-PK-ALPHA) (PROTEIN KINASE B) (PKB) (C-AKT) |
| P15056 | B-RAF PROTO-ONCOGENE SERINE/THREONINE-PROTEIN KINASE (P94) (V-RAF MURINE SARCOMA VIRAL ONCOGENE HOMOLOG B1) |
| P10398 | A-RAF PROTO-ONCOGENE SERINE/THREONINE-PROTEIN KINASE (ONCOGENE PKS2) |
| P14618 | PYRUVATE KINASE, M1 ISOZYME (PYRUVATE KINASE MUSCLE ISOZYME) (CYTOSOLIC THYROID HORMONE-BINDING PROTEIN) (CTHBP) (THBP1) |
| Q07002 | SERINE/THREONINE PROTEIN KINASE PCTAIRE-3 |
| Q00537 | SERINE/THREONINE-PROTEIN KINASE PCTAIRE-2 |
| P11801 | PUTATIVE SERINE/THREONINE-PROTEIN KINASE H1 (PSK-H1) |
| P11800 | PUTATIVE SERINE/THREONINE-PROTEIN KINASE PSK-C3 |
| P07557 | PKS PROTO-ONCOGENE SERINE/THREONINE-PROTEIN KINASE (ONCOGENE PKS1) |
| Q05513 | PROTEIN KINASE C, ZETA TYPE (NPKC-ZETA) |
| Q04759 | PROTEIN KINASE C, THETA TYPE (NPKC-THETA) |
| O94806 | PROTEIN KINASE C, NU TYPE (NPKC-NU) (PROTEIN KINASE EPK2) |
| Q15139 | PROTEIN KINASE C, MU TYPE (NPKC-MU) |
| P24723 | PROTEIN KINASE C, ETA TYPE (NPKC-ETA) (PKC-L) |
| P41743 | PROTEIN KINASE C, IOTA TYPE (NPKC-IOTA) |
| P05129 | PROTEIN KINASE C, GAMMA TYPE (PKC-GAMMA) |
| Q02156 | PROTEIN KINASE C, EPSILON TYPE (NPKC-EPSILON) |
| Q05655 | PROTEIN KINASE C, DELTA TYPE (NPKC-DELTA) |
| P17252 | PROTEIN KINASE C, ALPHA TYPE (PKC-ALPHA) |
| P05127 | PROTEIN KINASE C, BETA-II TYPE (PKC-BETA-2) |
| P05771 | PROTEIN KINASE C, BETA-I TYPE (PKC-BETA-1) |
| P27448 | PUTATIVE SERINE/THREONINE-PROTEIN KINASE P78 |
| P19525 | INTERFERON-INDUCED, DOUBLE-STRANDED RNA-ACTIVATED PROTEIN KINASE (INTERFERON-INDUCIBLE RNA-DEPENDENT PROTEIN KINASE) (P68 KINASE) (P1/EIF-2A PROTEIN KINASE) |
| P21127 | GALACTOSYLTRANSFERASE ASSOCIATED PROTEIN KINASE P58/GTA (CELL DIVISION CYCLE 2-LIKE 1) (CLK-1) (P58 CLK-1) |
| P00540 | PROTO-ONCOGENE SERINE/THREONINE-PROTEIN KINASE MOS (C-MOS) |
| Q15746 | MYOSIN LIGHT CHAIN KINASE, SMOOTH MUSCLE AND NON-MUSCLE ISOZYMES (MLCK) [CONTAINS: TELOKIN (KINASE RELATED PROTEIN) (KRP)] |
| P29376 | LEUKOCYTE TYROSINE KINASE RECEPTOR PRECURSOR (PROTEIN TYROSINE KINASE-1) |
| P10721 | MAST/STEM CELL GROWTH FACTOR RECEPTOR PRECURSOR (SCFR) (PROTO-ONCOGENE TYROSINE-PROTEIN KINASE KIT) (C-KIT) (CD117 ANTIGEN) |
| Q00532 | SERINE/THREONINE-PROTEIN KINASE KKIALRE (CYCLIN-DEPENDENT KINASE-LIKE 1) |
| P37023 | SERINE/THREONINE-PROTEIN KINASE RECEPTOR R3 PRECURSOR (SKR3) (ACTIVIN RECEPTOR-LIKE KINASE 1) (ALK-1) (TGF-B SUPERFAMILY RECEPTOR TYPE I) (TSR-I) |
| 075838 | KINASE INTERACTING PROTEIN 2 (KIP 2) |
| Q99828 | DNA-PKCS INTERACTING PROTEIN (KINASE INTERACTING PROTEIN) (KIP) (CALCIUM AND INTEGRIN-BINDING PROTEIN) (CIB) (SNK INTERACTING PROTEIN 2-28) (SIP2-28) |
| P14619 | CGMP-DEPENDENT PROTEIN KINASE 1, BETA ISOZYME (CGK 1 BETA) (CGKI-BETA) |
| Q13976 | CGMP-DEPENDENT PROTEIN KINASE 1, ALPHA ISOZYME (CGK 1 ALPHA) (CGKI-ALPHA) |
| Q13237 | CGMP-DEPENDENT PROTEIN KINASE 2 (CGK 2) (CGKII) (TYPE II CGMP-DEPENDENT PROTEIN KINASE) |
| Q13555 | CALCIUM/CALMODULIN-DEPENDENT PROTEIN KINASE TYPE II GAMMA CHAIN (CAM-KINASE II GAMMA CHAIN) (CAMK-II, GAMMA SUBUNIT) |
| Q13557 | CALCIUM/CALMODULIN-DEPENDENT PROTEIN KINASE TYPE II DELTA CHAIN (CAM-KINASE II DELTA CHAIN) (CAMK-II, DELTA SUBUNIT) |
| Q13554 | CALCIUM/CALMODULIN-DEPENDENT PROTEIN KINASE TYPE II BETA CHAIN (CAM-KINASE II BETA CHAIN) (CAMK-II, BETA SUBUNIT) |
| Q16566 | CALCIUM/CALMODULIN-DEPENDENT PROTEIN KINASE TYPE IV CATALYTIC CHAIN (CAM KINASE-GR) (CAMK IV) [CONTAINS: CALSPERMIN] |
| Q14012 | CALCIUM/CALMODULIN-DEPENDENT PROTEIN KINASE TYPE I (CAM KINASE I) |
| P22612 | CAMP-DEPENDENT PROTEIN KINASE, GAMMA-CATALYTIC SUBUNIT (PKA C-GAMMA) |
| P22694 | CAMP-DEPENDENT PROTEIN KINASE, BETA-CATALYTIC SUBUNIT (PKA C-BETA) |
| P17612 | CAMP-DEPENDENT PROTEIN KINASE, ALPHA-CATALYTIC SUBUNIT (PKA C-ALPHA) |
| P31323 | CAMP-DEPENDENT PROTEIN KINASE TYPE II-BETA REGULATORY CHAIN |
| P13861 | CAMP-DEPENDENT PROTEIN KINASE TYPE II-ALPHA REGULATORY CHAIN |
| P31321 | CAMP-DEPENDENT PROTEIN KINASE TYPE I-BETA REGULATORY CHAIN |
| P10644 | CAMP-DEPENDENT PROTEIN KINASE TYPE I-ALPHA REGULATORY CHAIN (TISSUE-SPECIFIC EXTINGUISHER-1) (TSE1) |
| Q9UBSO | RIBOSOMAL PROTEIN S6 KINASE BETA 2 (S6K-BETA 2) (70 KDA RIBOSOMAL PROTEIN S6 KINASE 2) (P70-S6KB) (P70 RIBOSOMAL S6 KINASE BETA) (P70 S6KBETA) (S6K2) (S6 KINASE-RELATED KINASE) (SRK) (SERINE/THREONINE-PROTEIN KINASE 14 BETA) |
| P23443 | RIBOSOMAL PROTEIN S6 KINASE (S6K) (P70-S6K) |
| Q9UK32 | RIBOSOMAL PROTEIN S6 KINASE ALPHA 6 (S6K-ALPHA 6) (90 KDA RIBOSOMAL PROTEIN S6 KINASE 6) (P90-RSK 6) (RIBOSOMAL S6 KINASE 4) (RSK-4) (PP90RSK4) |
| P51812 | RIBOSOMAL PROTEIN S6 KINASE ALPHA 3 (S6K-ALPHA 3) (90 KDA RIBOSOMAL PROTEIN S6 KINASE 3) (P90-RSK 3) (RIBOSOMAL S6 KINASE 2) (RSK-2) (PP90RSK2) (INSULIN-STIMULATED PROTEIN KINASE 1) (ISPK-1) |
| Q15349 | RIBOSOMAL PROTEIN S6 KINASE ALPHA 2 (S6K-ALPHA 2) (90 KDA RIBOSOMAL PROTEIN S6 KINASE 2) (P90-RSK 2) (RIBOSOMAL S6 KINASE 3) (RSK-3) (PP90RSK3) |
| Q15418 | RIBOSOMAL PROTEIN S6 KINASE ALPHA 1 (S6K-ALPHA 1) (90 KDA RIBOSOMAL PROTEIN S6 KINASE 1) (P90-RSK 1) (RIBOSOMAL S6 KINASE 1) (RSK-1) (PP90RSK1) |
| P52333 | TYROSINE-PROTEIN KINASE JAK3 (JANUS KINASE 3) (JAK-3) (LEUKOCYTE JANUS KINASE) (L-JAK) |
| P23458 | TYROSINE-PROTEIN KINASE JAK1 (JANUS KINASE 1) (JAK-1) |
| Q08881 | TYROSINE-PROTEIN KINASE ITK/TSK (T-CELL-SPECIFIC KINASE) (TYROSINE-PROTEIN KINASE LYK) (KINASE EMT) |
| | Q9Y2B9 CAMP-DEPENDENT PROTEIN KINASE INHIBITOR, GAMMA FORM (PKI-GAMMA) |
| | P04541 CAMP-DEPENDENT PROTEIN KINASE INHIBITOR, MUSCLE/BRAIN FORM (PKI-ALPHA) |
| P57043 | INTEGRIN-LINKED PROTEIN KINASE 2 (ILK-2) |
| Q13418 | INTEGRIN-LINKED PROTEIN KINASE 1 (ILK-1) (59 KDA SERINE/THREONINE PROTEIN KINASE) (P59ILK) |
| O95163 | IKAPPAB KINASE COMPLEX-ASSOCIATED PROTEIN (IKK COMPLEX-ASSOCIATED PROTEIN) (P150) |
| P57058 | HORMONALLY UPREGULATED NEU TUMOR-ASSOCIATED KINASE (SER)NE/THREONINE PROTEIN KINASE MAK-V) (B19) |
| Q9UJY1 | SMALL STRESS PROTEIN-LIKE PROTEIN HSP22 (E2IG1) (PROTEIN KINASE H11) |
| Q9H2X6 | HOMEODOMAIN-INTERACTING PROTEIN KINASE 2 |
| P08631 | TYROSINE-PROTEIN KINASE HCK (P59-HCK AND P60-HCK) (HEMOPOIETIC CELL KINASE) |
| P43250 | G PROTEIN-COUPLED RECEPTOR KINASE GRK6 |
| P34947 | G PROTEIN-COUPLED RECEPTOR KINASE GRK5 |
| P32298 | G PROTEIN-COUPLED RECEPTOR KINASE GRK4 (ITI1) |
| | Q14397 GLUCOKINASE REGULATORY PROTEIN (GLUCOKINASE REGULATOR) |
| P14314 | PROTEIN KINASE C SUBSTRATE, 80 KD PROTEIN, HEAVY CHAIN (PKCSH) (80K-H PROTEIN) |
| P06241 | PROTO-ONCOGENE TYROSINE-PROTEIN KINASE FYN (P59-FYN) (SYN) (SLK) |
| P42685 | TYROSINE-PROTEIN KINASE FRK (NUCLEAR TYROSINE PROTEIN KINASE RAK) |
| Q9HA64 | HYPOTHETICAL FRUCTOSAMINE KINASE-LIKE PROTEIN FLJ12171/DKFZP564D202 |
| P36888 | FL CYTOKINE RECEPTOR PRECURSOR (TYROSINE-PROTEIN KINASE RECEPTOR FLT3) (STEM CELL TYROSINE KINASE 1) (STK-1) (CD135 ANTIGEN) |
| P09769 | PROTO-ONCOGENE TYROSINE-PROTEIN KINASE FGR (P55-FGR) (C-FGR) |
| P07332 | PROTO-ONCOGENE TYROSINE-PROTEIN KINASE FES/FPS (C-FES) |
| P16591 | PROTO-ONCOGENE TYROSINE-PROTEIN KINASE FER (P94-FER) (C-FER) |
| Q05397 | FOCAL ADHESION KINASE 1 (FADK 1) (PP125FAK) (PROTEIN-TYROSINE KINASE 2) |
| O15197 | EPHRIN TYPE-B RECEPTOR 6 PRECURSOR (TYROSINE-PROTEIN KINASE-DEFECTIVE RECEPTOR EPH-6) (HEP) |
| P54760 | EPHRIN TYPE-B RECEPTOR 4 PRECURSOR (TYROSINE-PROTEIN KINASE RECEPTOR HTK) |
| Q15303 | ERBB-4 RECEPTOR PROTEIN-TYROSINE KINASE PRECURSOR (P180ERBB4) (TYROSINE KINASE-TYPE CELL SURFACE RECEPTOR HER4) |
| P21860 | ERBB-3 RECEPTOR PROTEIN-TYROSINE KINASE PRECURSOR (TYROSINE KINASE-TYPE CELL SURFACE RECEPTOR HER3) |
| P04626 | RECEPTOR PROTEIN-TYROSINE KINASE ERBB-2 PRECURSOR (P185ERBB2) (NEU PROTO-ONCOGENE) (C-ERBB-2) (TYROSINE KINASE-TYPE CELL SURFACE RECEPTOR HER2) (MLN 19) |
| P54753 | EPHRIN TYPE-B RECEPTOR 3 PRECURSOR (TYROSINE-PROTEIN KINASE RECEPTOR HEK-2) |
| P29323 | EPHRIN TYPE-B RECEPTOR 2 PRECURSOR (TYROSINE-PROTEIN KINASE RECEPTOR EPH-3) (DRT) (RECEPTOR PROTEIN-TYROSINE KINASE HEK5) (ERK) |
| P54762 | EPHRIN TYPE-B RECEPTOR 1 PRECURSOR (TYROSINE-PROTEIN KINASE RECEPTOR EPH-2) (NET) (HEK6) (ELK) |
| P29322 | EPHRIN TYPE-A RECEPTOR 8 (TYROSINE-PROTEIN KINASE RECEPTOR EEK) (EPH-AND ELK-RELATED KINASE) (HEK3) |
| Q15375 | EPHRIN TYPE-A RECEPTOR 7 PRECURSOR (TYROSINE-PROTEIN KINASE RECEPTOR EHK-3) (EPH HOMOLOGY KINASE-3) (RECEPTOR PROTEIN-TYROSINE KINASE HEK11) |
| P54756 | EPHRIN TYPE-A RECEPTOR 5 PRECURSOR (TYROSINE-PROTEIN KINASE RECEPTOR EHK-1) (EPH HOMOLOGY KINASE-1) (RECEPTOR PROTEIN-TYROSINE KINASE HEK7) |
| P54764 | EPHRIN TYPE-A RECEPTOR 4 PRECURSOR (TYROSINE-PROTEIN KINASE RECEPTOR SEK) (RECEPTOR PROTEIN-TYROSINE KINASE HEK8) |
| P29320 | EPHRIN TYPE-A RECEPTOR 3 PRECURSOR (TYROSINE-PROTEIN KINASE RECEPTOR ETK1) (HEK) (HEK4) |
| P29317 | EPHRIN TYPE-A RECEPTOR 2 PRECURSOR (TYROSINE-PROTEIN KINASE RECEPTOR ECK) (EPITHELIAL CELL KINASE) |
| P21709 | EPHRIN TYPE-A RECEPTOR 1 PRECURSOR (TYROSINE-PROTEIN KINASE RECEPTOR EPH) |
| Q9NVF9 | ETHANOLAMINE KINASE-LIKE PROTEIN EKI2 (FLJ10761) |
| P20827 | EPHRIN-A1 PRECURSOR (EPH-RELATED RECEPTOR TYROSINE KINASE LIGAND 1) (LERK-1) (IMMEDIATE EARLY RESPONSE PROTEIN B61) (TUMOR NECROSIS FACTOR, ALPHA-INDUCED PROTEIN 4) |
| Q99956 | DUAL SPECIFICITY PROTEIN PHOSPHATASE 9 (MITOGEN-ACTIVATED PROTEIN KINASE PHOSPHATASE 4) (MAP KINASE PHOSPHATASE 4) (MKP-4) |
| Q16828 | DUAL SPECIFICITY PROTEIN PHOSPHATASE 6 (MITOGEN-ACTIVATED PROTEIN KINASE PHOSPHATASE 3) (MAP KINASE PHOSPHATASE 3) (MKP-3) (DUAL SPECIFICITY PROTEIN PHOSPHATASE PYST1) |
| Q9Y463 | DUAL-SPECIFICITY TYROSINE-PHOSPHORYLATION REGULATED KINASE 1B (MIRK PROTEIN KINASE) |
| Q13627 | DUAL-SPECIFICITY TYROSINE-PHOSPHORYLATION REGULATED KINASE 1A (PROTEIN KINASE MINIBRAIN HOMOLOG) (MNBH) (HP86) (DUAL SPECIFICITY YAK1-RELATED KINASE) |
| P28562 | DUAL SPECIFICITY PROTEIN PHOSPHATASE 1 (MAP KINASE PHOSPHATASE-1) (MKP-1) (PROTEIN-TYROSINE PHOSPHATASE CL100) (DUAL SPECIFICITY PROTEIN PHOSPHATASE HVH1) |
| Q09013 | MYOTONIN-PROTEIN KINASE (MYOTONIC DYSTROPHY PROTEIN KINASE) (MDPK) (DM-KINASE) (DMK) (DMPK) (MT-PK) |
| Q16832 | DISCOIDIN DOMAIN RECEPTOR 2 PRECURSOR (RECEPTOR PROTEIN-TYROSINE KINASE TKT) (TYROSINE-PROTEIN KINASE TYRO 10) (NEUROTROPHIC TYROSINE KINASE, RECEPTOR-RELATED 3) |
| Q08345 | EPITHELIAL DISCOIDIN DOMAIN RECEPTOR 1 PRECURSOR (TYROSINE-PROTEIN KINASE CAK) (CELL ADHESION KINASE) (TYROSINE KINASE DDR) (DISCOIDIN RECEPTOR TYROSINE KINASE) (TRK E) (PROTEIN-TYROSINE KINASE RTK 6) |
| P53355 | DEATH-ASSOCIATED PROTEIN KINASE 1 (DAP KINASE 1) |
| O15075 | SERINE/THREONINE-PROTEIN KINASE DCAMKL1 (DOUBLECORTIN-LIKE AND CAM KINASE-LIKE 1) |
| P41240 | TYROSINE-PROTEIN KINASE CSK (C-SRC KINASE) (PROTEIN-TYROSINE KINASE CYL) |
| Q9NYV4 | CELL DIVISION CYCLE 2-RELATED PROTEIN KINASE 7 (CDC2-RELATED PROTEIN KINASE 7) (CRKRS) |
| Q9H4B4 | CYTOKINE-INDUCIBLE SERINE/THREONINE-PROTEIN KINASE (FGF-INDUCIBLE KINASE) (PROLIFERATION-RELATED KINASE) |
| P49761 | PROTEIN KINASE CLK3 |
| P49760 | PROTEIN KINASE CLK2 |
| P49759 | PROTEIN KINASE CLK1 (CLK) |
| O96017 | SERINE/THREONINE-PROTEIN KINASE CHK2 (CDS1) |
| O14757 | SERINE/THREONINE-PROTEIN KINASE CHK1 |
| Q16667 | CYCLIN-DEPENDENT KINASE INHIBITOR 3 (CDK2-ASSOCIATED DUAL SPECIFICITY PHOSPHATASE) (KINASE ASSOCIATED PHOSPHATASE) (CYCLIN-DEPENDENT KINASE INTERACTING PROTEIN 2) (CYCLIN-DEPENDENT KINASE INTERACTOR 1) |
| Q14004 | CELL DIVISION CYCLE 2-LIKE PROTEIN KINASE 5 (CHOLINESTERASE-RELATED CELL DIVISION CONTROLLER) (CDC2-RELATED PROTEIN KINASE 5) |
| Q15131 | CELL DIVISION PROTEIN KINASE 10 (SERINE/THREONINE-PROTEIN KINASE PISSLRE) |
| P50750 | CELL DIVISION PROTEIN KINASE 9 (SERINE/THREONINE-PROTEIN KINASE PITALRE) (C-2K) |
| P49336 | CELL DIVISION PROTEIN KINASE 8 (PROTEIN KINASE K35) |
| P50613 | CELL DIVISION PROTEIN KINASE 7 (CDK-ACTIVATING KINASE) (CAK) (39 KDA PROTEIN KINASE) (P39 MO15) (STK1) (CAK1) |
| Q00534 | CELL DIVISION PROTEIN KINASE 6 (SERINE/THREONINE-PROTEIN KINASE PLSTIRE) |
| Q00535 | CELL DIVISION PROTEIN KINASE 5 (TAU PROTEIN KINASE II CATALYTIC SUBUNIT) (TPKII CATALYTIC SUBUNIT) (SERINE/THREONINE-PROTEIN KINASE PSSALRE) |
| Q00526 | CELL DIVISION PROTEIN KINASE 3 |
| P24941 | CELL DIVISION PROTEIN KINASE 2 (P33 PROTEIN KINASE) |
| O14519 | CYCLIN-DEPENDENT KINASE 2-ASSOCIATED PROTEIN 1 (CDK2-ASSOCIATED PROTEIN 1) (PUTATIVE ORAL CANCER SUPPRESSOR) (DELETED IN ORAL CANCER-1) (DOC-1) |
| O00311 | CELL DIVISION CYCLE 7-RELATED PROTEIN KINASE (CDC7-RELATED KINASE) (HSCDC7) (HUCDC7) |
| Q15078 | CYCLIN-DEPENDENT KINASE 5 ACTIVATOR 1 PRECURSOR (CDK5 ACTIVATOR 1) (CYCLIN-DEPENDENT KINASE 5 REGULATORY SUBUNIT 1) (TAU PROTEIN KINASE II 23 KDA SUBUNIT) (TPKII REGULATORY SUBUNIT) (P23) (P25) (P35) |
| P06493 | CELL DIVISION CONTROL PROTEIN 2 HOMOLOG (P34 PROTEIN KINASE) (CYCLIN-DEPENDENT KINASE 1) (CDK1) |
| Q9UHJ6 | CARBOHYDRATE KINASE-LIKE PROTEIN |
| O43683 | MITOTIC CHECKPOINT SERINE/THREONINE-PROTEIN KINASE BUB1 (HBUB1) (BUB1A) |
| O60566 | MITOTIC CHECKPOINT SERINE/THREONINE-PROTEIN KINASE BUB1 BETA (HBUBR1) (MAD3/BUB1-RELATED PROTEIN KINASE) (MITOTIC CHECKPOINT KINASE MAD3L) |
| Q06187 | TYROSINE-PROTEIN KINASE BTK (BRUTON'S TYROSINE KINASE) (AGAMMAGLOBULINAEMIA TYROSINE KINASE) (ATK) (B CELL PROGENITOR KINASE) (BPK) |
| P51813 | CYTOPLASMIC TYROSINE-PROTEIN KINASE BMX (BONE MARROW KINASE BMX) (EPITHELIAL AND ENDOTHELIAL TYROSINE KINASE) (ETK) (NTK38) |
| P36894 | BONE MORPHOGENETIC PROTEIN RECEPTOR TYPE IA PRECURSOR (SERINE/THREONINE-PROTEIN KINASE RECEPTOR R5) (SKR5) (ACTIVIN RECEPTOR-LIKE KINASE 3) (ALK-3) |
| P51451 | TYROSINE-PROTEIN KINASE BLK (B LYMPHOCYTE KINASE) (P55-BLK) |
| Q04771 | ACTIVIN RECEPTOR TYPE I PRECURSOR (ACTR-I) (SERINE/THREONINE-PROTEIN KINASE RECEPTOR R1) (SKR1) (ACTIVIN RECEPTOR-LIKE KINASE 2) (ALK-2) (TGF-B SUPERFAMILY RECEPTOR TYPE I) (TSR-I) |
| Q13315 | SERINE-PROTEIN KINASE ATM (ATAXIA TELANGIECTASIA MUTATED) (A-T, MUTATED) |
| P35626 | BETA-ADRENERGIC RECEPTOR KINASE 2 (BETA-ARK-2) (G-PROTEIN COUPLED RECEPTOR KINASE 3) |
| P25098 | BETA-ADRENERGIC RECEPTOR KINASE 1 (BETA-ARK-1) (G-PROTEIN COUPLED RECEPTOR KINASE 2) |
| P57078 | SERINE/THREONINE-PROTEIN KINASE ANKRD3 (ANKYRIN REPEAT DOMAIN PROTEIN 3) |
| Q9Y243 | RAC-GAMMA SERINE/THREONINE PROTEIN KINASE (RAC-PK-GAMMA) (PROTEIN KINASE AKT-3) (PROTEIN KINASE B, GAMMA) (PKB GAMMA) |
| P31751 | RAC-BETA SERINE/THREONINE PROTEIN KINASE (RAC-PK-BETA) (PROTEIN KINASE AKT-2) (PROTEIN KINASE B, BETA) (PKB BETA) |
| Q02952 | A-KINASE ANCHOR PROTEIN 12 (A-KINASE ANCHOR PROTEIN 250 KDA) (AKAP 250) (MYASTHENIA GRAVIS AUTOANTIGEN GRAVIN) |
| Q99996 | A KINASE ANCHOR PROTEIN 9 (PROTEIN KINASE A ANCHORING PROTEIN 9) (PRKA9) (A-KINASE ANCHOR PROTEIN 450 KDA) (AKAP 450) (A-KINASE ANCHOR PROTEIN 350 KDA) (AKAP 350) (HGAKAP 350) (AKAP 120 LIKE PROTEIN) (HYPERION PROTEIN) (YOTIAO PROTEIN) (CENTROS |
| O43823 | A-KINASE ANCHOR PROTEIN 8 (A-KINASE ANCHOR PROTEIN 95 KDA) (AKAP 95) |
| O43687 | A-KINASE ANCHOR PROTEIN 7 (A-KINASE ANCHOR PROTEIN 9 KDA) |
| Q13023 | A KINASE ANCHOR PROTEIN 6 (PROTEIN KINASE A ANCHORING PROTEIN 6) (PRKA6) (A-KINASE ANCHOR PROTEIN 100 KDA) (AKAP 100) (MAKAP) |
| P24588 | A-KINASE ANCHOR PROTEIN 5 (A-KINASE ANCHOR PROTEIN 79 KDA) (AKAP 79) (CAMP-DEPENDENT PROTEIN KINASE REGULATORY SUBUNIT II HIGH AFFINITY BINDING PROTEIN) (H21) |
| O75969 | A KINASE ANCHOR PROTEIN 3 (PROTEIN KINASE A ANCHORING PROTEIN 3) (PRKA3) (A-KINASE ANCHOR PROTEIN 110 KDA) (AKAP 110) (SPERM OOCYTE BINDING PROTEIN) (FIBROUSHEATHIN I) (FIBROUS SHEATH PROTEIN OF 95 KDA) (FSP95) |
| Q9Y2D5 | A KINASE ANCHOR PROTEIN 2 (PROTEIN KINASE A ANCHORING PROTEIN 2) (PRKA2) |
| Q92667 | A KINASE ANCHOR PROTEIN 1, MITOCHONDRIAL PRECURSOR (PROTEIN KINASE A ANCHORING PROTEIN 1) (PRKA1) (A-KINASE ANCHOR PROTEIN 149 KDA) (AKAP 149) (DUAL SPECIFICITY A-KINASE ANCHORING PROTEIN 1) (D-AKAP-1) (SPERMATID A-KINASE ANCHOR PROTEIN 84) (S- |
| Q9UKA4 | A KINASE ANCHOR PROTEIN 11 (PROTEIN KINASE A ANCHORING PROTEIN 11) (PRKA11) (A KINASE ANCHOR PROTEIN 220 KDA) (AKAP 220) (HAKAP220) |
| O43572 | A KINASE ANCHOR PROTEIN 10, MITOCHONDRIAL PRECURSOR (PROTEIN KINASE A ANCHORING PROTEIN 10) (PRKA10) (DUAL SPECIFICITY. A-KINASE ANCHORING PROTEIN 2) (D-AKAP-2) |
| P42684 | TYROSINE-PROTEIN KINASE ABL2 (TYROSINE KINASE ARG) |
| P00519 | PROTO-ONCOGENE TYROSINE-PROTEIN KINASE ABL1 (P150) (C-ABL) |
| Q9UG19 | 5'-AMP-ACTIVATED PROTEIN KINASE, GAMMA-3 SUBUNIT (AMPK GAMMA-3 CHAIN) (AMPK GAMMA3) |
| Q9UGJ0 | 5'-AMP-ACTIVATED PROTEIN KINASE, GAMMA-2 SUBUNIT (AMPK GAMMA-2 CHAIN) (AMPK GAMMA2) (H91620P) |
| P54619 | 5'-AMP-ACTIVATED PROTEIN KINASE, GAMMA-1 SUBUNIT (AMPK GAMMA-1 CHAIN) (AMPKG) |
| O43741 | 5'-AMP-ACTIVATED PROTEIN KINASE, BETA-2 SUBUNIT (AMPK BETA-2 CHAIN) |
| Q9Y478 | 5'-AMP-ACTIVATED PROTEIN KINASE, BETA-1 SUBUNIT (AMPK BETA-1 CHAIN) (AMPKB) |
| P54646 | 5'-AMP-ACTIVATED PROTEIN KINASE, CATALYTIC ALPHA-2 CHAIN (AMPK ALPHA-2 CHAIN) |
| Q13131 | 5'-AMP-ACTIVATED PROTEIN KINASE, CATALYTIC ALPHA-1 CHAIN (AMPK ALPHA-1 CHAIN) |
| P42655 | 14-3-3 PROTEIN EPSILON (MITOCHONDRIAL IMPORT STIMULATION FACTOR SUBUNIT) (PROTEIN KINASE C INHIBITOR PROTEIN-1) (KCIP-1) (14-3-3E) |
| P31946 | 14-3-3 PROTEIN BETA/ALPHA (PROTEIN KINASE C INHIBITOR PROTEIN-1) (KCIP-1) (PROTEIN 1054) |

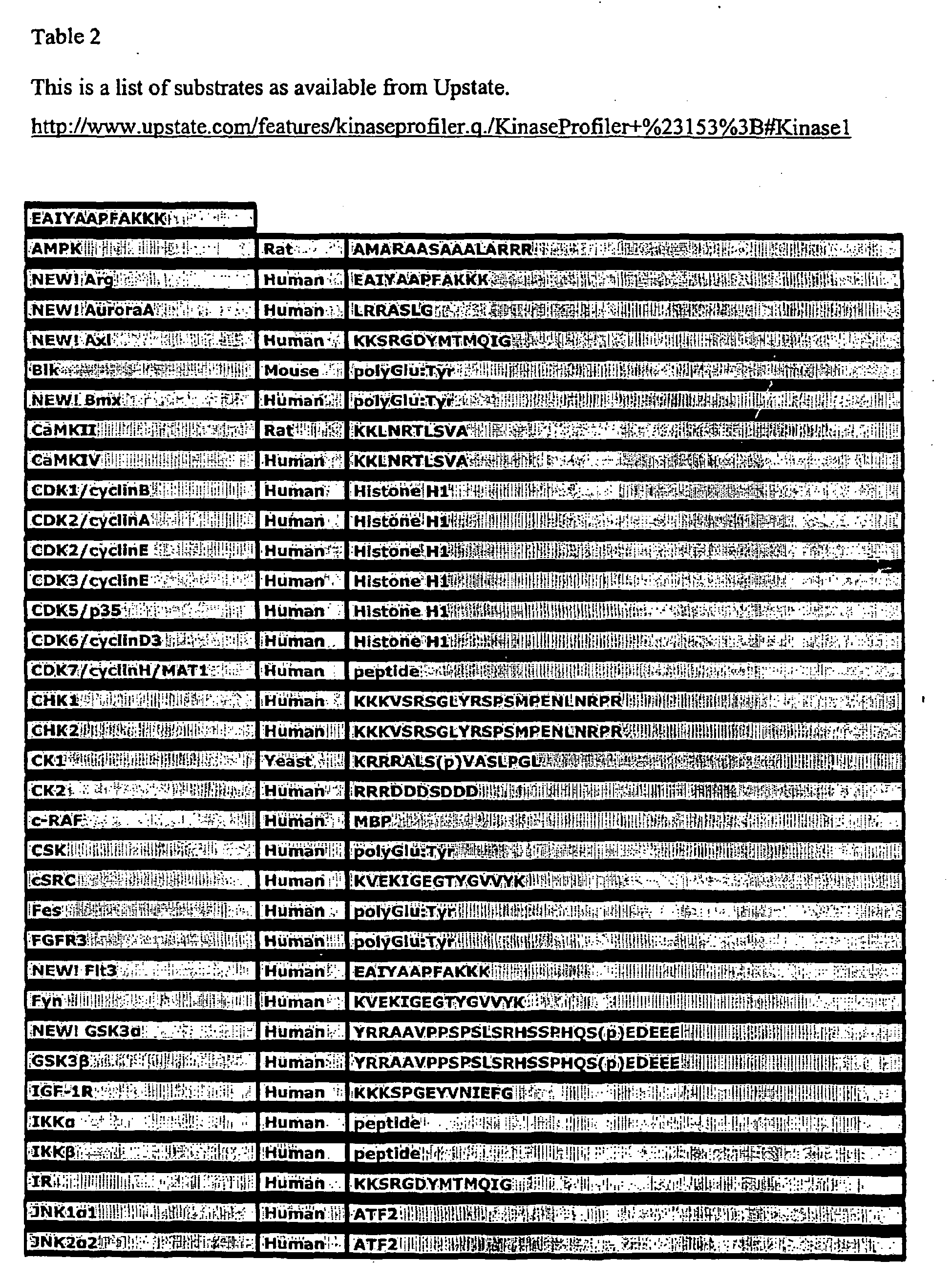

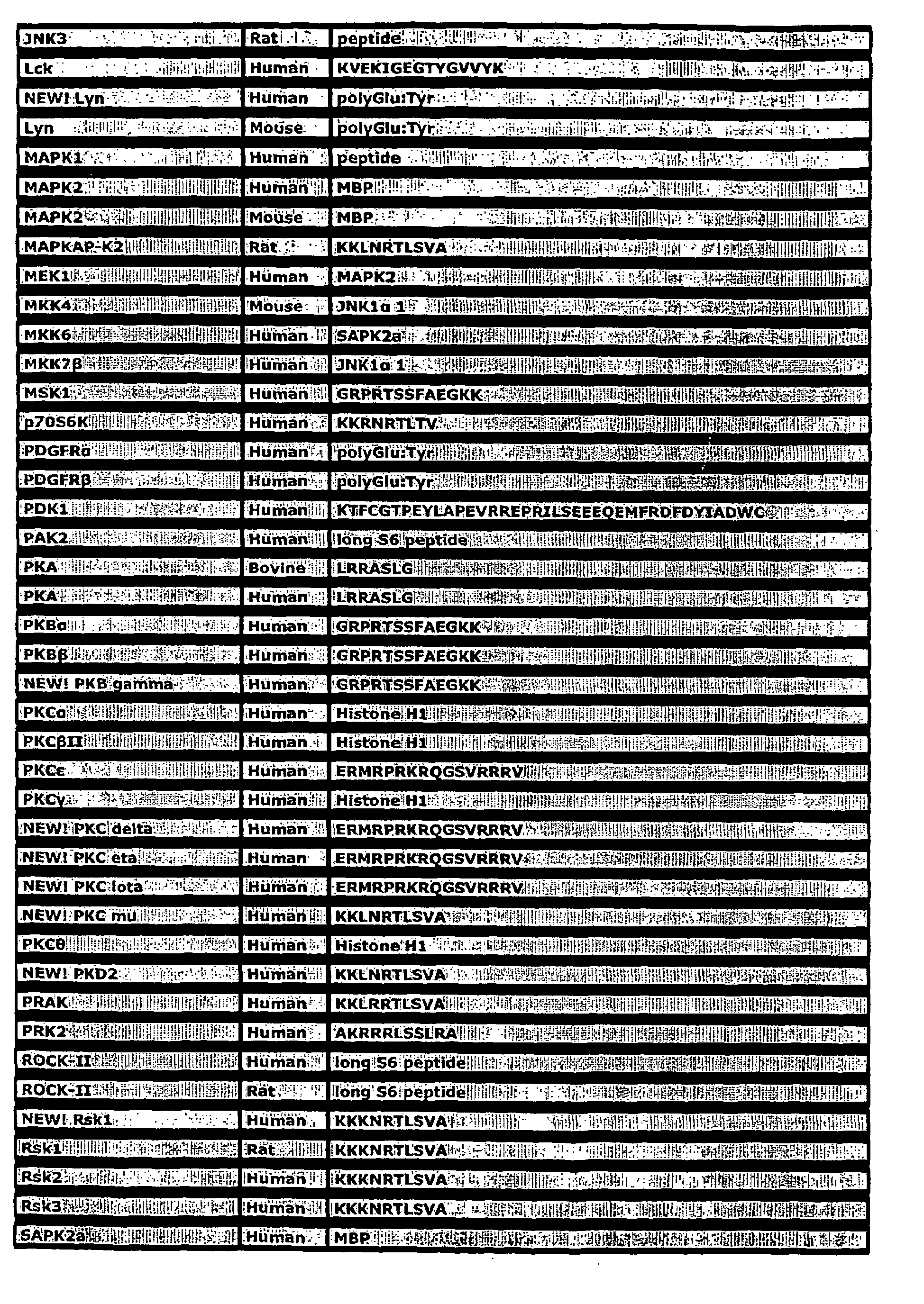

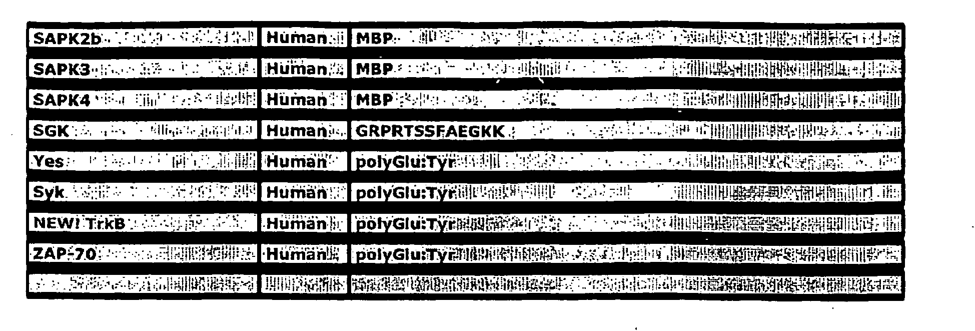

## Claims

1. A method of determining the activity of an enzyme, or the effect a test compound has on the activity of the enzyme, by using matrix-assisted laser desorption-ionisation mass spectrometry or desorption-ionisation on silicon mass spectrometry comprising:
(i) providing a probe carrying an immobilised enzyme;
(ii) optionally introducing the test compound;
(iii) introducing one or more reactants to the immobilised enzyme for a time, and in a form sufficient for a reaction to take place;
(iv) optionally drying the probe;
(v) subjecting the probe to matrix-assisted laser desorption-ionisation mass spectrometry or desorption-ionisation on silicon mass spectrometry;
(vi) determining the activity of the enzyme, or the effect the test compound had on the activity of the enzyme, by detecting the presence and/or absence of one or more products and/or the one or more reactants;
**characterised in that** a layer resistant to non-specific protein binding is provided on the probe surface,
and wherein the mass spectrometer is a laser desorption ionisation mass spectrometer, preferably a MALDI mass spectrometer.

2. A method according to claim 1, wherein said layer resistant to non-specific protein binding comprises protein repellent molecules such as polyethylene glycol molecules, which protein repellent molecules are immobilised on the probe surface.

3. A method according to claim 1 or claim 2, wherein the enzyme is a kinase such as a serine kinase, tyrosine kinase or threonine kinase, an oxidoreductase, a transferase, a hydrolase, a lyase, a ligase, a carboxylase, an esterase, a phosphodiesterase, a protein phosphatase such as a tyrosine phosphatase, a G-protein coupled receptor, an ATP-dependent chaperone, a cyclooxygenase, a cytochrome P450, a sialidase, a short-chain dehydrogenase, a short-chain reductase, or an isomerase.

4. A method according to any preceding claim for determining the activity of one or more kinases or the effect a test compound has on the activity of one or more kinases by using MALDI mass spectrometry.

5. A method as claimed in claim 4, wherein the one or more reactants comprise a phosphate donor, a phosphate acceptor and a divalent cation.

6. A method as claimed in claim 5, wherein the phosphate donor is a phosphorylated substrate and the phosphate acceptor is a nucleotide di phosphate (NDP).

7. A method as claimed in claim 5, wherein the phosphate donor is a nucleotide tri phosphate (NTP) and the phosphate acceptor is a substrate to be phosphorylated.

8. A method as claimed in claim 5, wherein the divalent cation is magnesium or manganese.

9. A method as claimed in claim 6 or claim 7, wherein the nucleotide di phosphate or tri phosphate is an adenine di or tri phosphate.

10. A method as claimed in any preceding claim, wherein the product is a nucleotide tri phosphate or a nucleotide di phosphate and its presence is detected.

11. A method as claimed in claim 10, wherein the nucleotide tri phosphate or nucleotide di phosphate are detected as [NDP]⁻ or [NTP]⁻ or as one or more adduct peaks thereof .

12. A method as claimed in claim 11, wherein the one or more adduct peaks are adduct peaks with a monovalent cation (M⁺).

13. A method as claimed in claim 12, wherein the one or more adduct peaks include: [ATP+M]⁻, [ATP+2M]⁻ and [ATP+3M]⁻ and/or [ADP+M]⁻, [ADP+2M]⁻, and [ADP+3M]⁻.

14. A method as claimed in any preceding claim, further comprising, between step (iv) and step (v), overlaying the probe with energy absorbing molecules.

15. A method as claimed in claim 14, wherein said energy absorbing molecules are deposited onto the probe surface in a non-aqueous solvent, followed by evaporation of the solvent.

16. A method as claimed in any preceding claim, wherein said probe carries more than one enzyme.

17. A method as claimed in any preceding claim, wherein in step (iii) said one or more reactants are provided in a low salt buffer.

18. A method as claimed in claim 17, wherein said low salt buffer is a semi-volatile buffer such as ammonium bicarbonate buffer.

19. A method as claimed in any of claims 1-16, wherein in step (iii) said one or more reactants are provided in a semi volatile buffer, and further comprising the step, after the reaction is finished, of removing the semi-volatile buffer.

20. A method as claimed in any preceding claim, wherein the enzymes are attached to the probe as fusion proteins, typically via a tag.

21. A method as claimed in any preceding claim, wherein said test compound is added before, after or with the one or more reactants to determine its effect on enzyme activity.

22. A method as claimed in any preceding claim, wherein the one or more reactants and the optional test compound are introduced to the immobilised enzyme as a droplet, such as a droplet having a volume of less than 1 microlitre.

## Patentansprüche

1. Verfahren zur Bestimmung der Aktivität eines Enzyms oder der Wirkung, die eine Testverbindung auf die Aktivität des Enzyms hat, durch Verwendung von Matrix-unterstützter Laser-Desorptions-lonisations-Massenspektrometrie oder von Massenspektrometrie mit Desorption-lonisation auf Silicium, aufweisend:
(i) Bereitstellen eines Aufnehmers, der ein immobilisiertes Enzym trägt;
(ii) optional Einführen der Testverbindung;
(iii) Einführen eines oder mehrerer Reaktionsmittel zu dem immobilisierten Enzym für eine Zeit und in einer Form, die ausreichend sind, dass eine Reaktion stattfindet;
(iv) optional Trocknen des Aufnehmers;
(v) Unterziehen des Aufnehmers einer Matrix-unterstützten Laser-Desorptions-lonisations-Massenspektrometrie oder einer Massenspektrometrie mit Desorption-Ionisation auf Silicium;
(vi) Bestimmen der Aktivität des Enzyms oder der Wirkung, die die Testverbindung auf die Aktivität des Enzyms hatte, durch Nachweisen des Vorliegens und/oder Fehlens eines oder mehrerer Produkte und/oder des einen oder der mehreren Reaktionsmittel;
**dadurch gekennzeichnet, dass** auf der Oberfläche des Aufnehmers eine gegen unspezifische Proteinbindung beständige Schicht vorgesehen wird,
und wobei das Massenspektrometer ein Laser-Desorptions-Ionisations-Massenspektrometer, bevorzugt ein MALDI-Massenspektrometer, ist.

2. Verfahren nach Anspruch 1, bei dem die gegen unspezifische Proteinbindung beständige Schicht Protein-Abstoßungsmoleküle wie Polyethylenglycol-Moleküle aufweist, wobei die Protein-Abstoßungsmoleküle auf der Aufnehmer-Oberfläche immobilisiert sind.

3. Verfahren nach Anspruch 1 oder Anspruch 2, bei dem das Enzym eine Kinase wie eine Serinkinase, Tyrosinkinase oder Threoninkinase, eine Oxidoreductase, eine Transferase, eine Hydrolase, eine Lyase, eine Ligase, eine Carboxylase, eine Esterase, eine Phosphodiesterase, eine Protein-Phosphatase wie eine Tyrosin-Phosphatase, ein G-Protein-gekoppelter Rezeptor, ein ATPabhängiges Chaperon, eine Cyclooxygenase, ein Cytochrom P450, eine Sialidase, eine kurzkettige Dehydrogenase, eine kurzkettige Reductase oder eine Isomerase ist.

4. Verfahren nach einem vorangehenden Anspruch zur Bestimmung der Aktivität einer oder mehrerer Kinasen oder der Wirkung, die eine Testverbindung auf die Aktivität einer oder mehrerer Kinasen hat, durch Verwendung von MALDI-Massenspektrometrie.

5. Verfahren wie in Anspruch 4 beansprucht, bei dem das eine oder die mehreren Reaktionsmittel einen Phosphat-Donor, einen Phosphat-Akzeptor und ein zweiwertiges Kation aufweisen.

6. Verfahren wie in Anspruch 5 beansprucht, bei dem der Phosphat-Donor ein phosphoryliertes Substrat ist und der Phosphat-Akzeptor ein Nucleotid-diphosphat (NDP) ist.

7. Verfahren wie in Anspruch 5 beansprucht, bei dem der Phosphat-Donor ein Nucleotid-triphosphat (NTP) ist und der Phosphat-Akzeptor ein zu phosphorylierendes Substrat ist.

8. Verfahren wie in Anspruch 5 beansprucht, bei dem das zweiwertige Kation Magnesium oder Mangan ist.

9. Verfahren wie in Anspruch 6 oder in Anspruch 7 beansprucht, bei dem das Nucleotid-diphosphat oder das Nucleotid-triphosphat ein Adenin-diphosphat oder ein Adenin-triphosphat ist.

10. Verfahren wie in einem vorangehenden Anspruch beansprucht, bei dem das Produkt ein Nucleotid-triphosphat oder ein Nucleotid-diphosphat ist und sein Vorliegen nachgewiesen wird.

11. Verfahren wie in Anspruch 10 beansprucht, bei dem das Nucleotid-triphosphat oder das Nucleotid-diphosphat als Peaks von [NDP]⁻ oder [NTP]⁻ oder eines oder mehrerer Addukte davon nachgewiesen wird.

12. Verfahren wie in Anspruch 11 beansprucht, bei dem der eine oder die mehreren Addukt-Peaks, Addukt-Peaks mit einem einwertigen Kation (M⁺) sind.

13. Verfahren wie in Anspruch 12 beansprucht, bei dem der eine oder die mehreren Addukt-Peaks umfassen: [ATPM]⁻, [ATPM₂]⁻ und [ATPM₃]⁻ und/oder [ADPM]⁻, [ADPM₂]⁻ und [ADPM₃]⁻.

14. Verfahren wie in einem vorangehenden Anspruch beansprucht, außerdem aufweisend, zwischen Schritt (iv) und Schritt (v), ein Überschichten des Aufnehmers mit Energie-absorbierenden Molekülen.

15. Verfahren wie in Anspruch 14 beansprucht, bei dem die Energie-absorbierenden Moleküle in einem nicht-wässrigen Lösungsmittel auf der Aufnehmer-Oberfläche abgeschieden werden, gefolgt von Verdampfen des Lösungsmittels.

16. Verfahren wie in einem vorangehenden Anspruch beansprucht, bei dem der Aufnehmer mehr als ein Enzym trägt.

17. Verfahren wie in einem vorangehenden Anspruch beansprucht, bei dem in Schritt (iii) das eine oder die mehreren Reaktionsmittel in einem salzarmen Puffer vorgesehen werden.

18. Verfahren wie in Anspruch 17 beansprucht, bei dem der salzarme Puffer ein halbflüchtiger Puffer wie ein Ammonium-bicarbonat-Puffer ist.

19. Verfahren wie in einem der Ansprüche 1 bis 16 beansprucht, bei dem in Schritt (iii) das eine oder die mehreren Reaktionsmittel in einem halbflüchtigen Puffer vorgesehen werden; und das außerdem den Schritt des Entfernens des halbflüchtigen Puffers, nachdem die Reaktion beendet ist, aufweist.

20. Verfahren wie in einem vorangehenden Anspruch beansprucht, bei dem die Enzyme als Fusionsproteine, typischerweise mittels eines Tags, an den Aufnehmer gebunden werden.

21. Verfahren wie in einem vorangehenden Anspruch beansprucht, bei dem die Testverbindung vor, nach oder mit dem einen oder den mehreren Reaktionsmitteln zugegeben wird, um ihre Wirkung auf die Enzym-Aktivität zu bestimmen.

22. Verfahren wie in einem vorangehenden Anspruch beansprucht, bei dem das eine oder die mehreren Reaktionsmittel und die optionale Testverbindung als ein Tropfen, wie ein Tropfen mit einem Volumen von weniger als einem Mikroliter, dem immobilisierten Enzym zugeführt werden.

## Revendications

1. Procédé de détermination de l'activité d'une enzyme, ou de l'effet qu'un composé de test a sur l'activité de l'enzyme, en utilisant la spectrométrie de masse à désorption-ionisation laser assistée par matrice ou la spectrométrie de masse à désorption-ionisation sur silicium comprenant les étapes consistant à :
(i) se procurer une sonde portant une enzyme immobilisée ;
(ii) introduire éventuellement le composé de test ;
(iii) introduire un ou plusieurs réactants au niveau de l'enzyme immobilisée pendant un certain temps et sous une forme suffisante pour qu'une réaction ait lieu ;
(iv) sécher éventuellement la sonde ;
(v) soumettre la sonde à une spectrométrie de masse à désorption-ionisation laser assistée par matrice ou une spectrométrie de masse à désorption-ionisation sur silicium ;
(vi) déterminer l'activité de l'enzyme, ou l'effet que le composé de test avait sur l'activité de l'enzyme, par détection de la présence et/ou de l'absence d'un ou plusieurs produits et/ou du ou des réactants ;
**caractérisé en ce qu'**une couche résistant à une liaison non spécifique à une protéine est disposée à la surface de la sonde,
et dans lequel le spectromètre de masse est un spectromètre de masse à désorption-ionisation laser, de préférence un spectromètre de masse MALDI.

2. Procédé selon la revendication 1, dans lequel ladite couche résistant à une liaison non spécifique à une protéine comprend des molécules repoussant les protéines, telles que des molécules de polyéthylèneglycol, lesquelles molécules repoussant les protéines sont immobilisées à la surface de la sonde.

3. Procédé selon la revendication 1 ou la revendication 2, dans lequel l'enzyme est une kinase telle qu'une sérine kinase, tyrosine kinase ou thréonine kinase, une oxydoréductase, une transférase, une hydrolase, une lyase, une ligase, une carboxylase, une estérase, une phosphodiestérase, une protéine phosphatase telle qu'une tyrosine phosphatase, un récepteur couplé à une protéine G, un chaperon ATP-dépendant, une cyclooxygénase, un cytochrome P450, une sialidase, une déshydrogénase à chaîne courte, une réductase à chaîne courte ou une isomérase.

4. Procédé selon l'une quelconque des revendications précédentes pour la détermination de l'activité d'une ou plusieurs kinases ou de l'effet qu'un composé de test a sur l'activité d'une ou plusieurs kinases par utilisation d'une spectrométrie de masse MALDI.

5. Procédé selon la revendication 4, dans lequel le ou les réactants comprennent un donneur de phosphate, un accepteur de phosphate et un cation divalent.

6. Procédé selon la revendication 5, dans lequel le donneur de phosphate est un substrat phosphorylé et l'accepteur de phosphate est un nucléotide diphosphate (NDP).

7. Procédé selon la revendication 5, dans lequel le donneur de phosphate est un nucléotide triphosphate (NTP) et l'accepteur de phosphate est un substrat à phosphoryler.

8. Procédé selon la revendication 5, dans lequel le cation divalent est le magnésium ou le manganèse.

9. Procédé selon la revendication 6 ou la revendication 7, dans lequel le nucléotide diphosphate ou triphosphate est une adénine di ou triphosphate.

10. Procédé selon l'une quelconque des revendications précédentes, dans lequel le produit est un nucléotide triphosphate ou un nucléotide diphosphate et sa présence est détectée.

11. Procédé selon la revendication 10, dans lequel le nucléotide triphosphate ou le nucléotide diphosphate est détecté en tant que [NDP]⁻ ou [NTP]⁻ ou en tant qu'un ou plusieurs de leurs pics d'adduit.

12. Procédé selon la revendication 11, dans lequel le ou les pics d'adduit sont des pics d'adduit avec un cation monovalent (M⁺).

13. Procédé selon la revendication 12, dans lequel le ou les pics d'adduit comprennent : [ATPM]⁻, [ATPM₂]⁻ et [ATPM₃]⁻ et/ou [ADPM]⁻, [ADPM₂]⁻ et [ADPM₃]⁻.

14. Procédé selon l'une quelconque des revendications précédentes, comprenant en outre, entre l'étape (iv) et l'étape (v), le recouvrement de la sonde avec des molécules absorbant de l'énergie.

15. Procédé selon la revendication 14, dans lequel lesdites molécules absorbant de l'énergie sont déposées à la surface de la sonde dans un solvant non aqueux, opération suivie d'une évaporation du solvant.

16. Procédé selon l'une quelconque des revendications précédentes, dans lequel ladite sonde porte plus d'une enzyme.

17. Procédé selon l'une quelconque des revendications précédentes, dans lequel dans l'étape (iii) le(s)dit(s) un ou plusieurs réactants sont mis en oeuvre dans un tampon à faible teneur en sel.

18. Procédé selon la revendication 17, dans lequel ledit tampon à faible teneur en sel est un tampon semi volatil tel qu'un tampon au bicarbonate d'ammonium.

19. Procédé selon l'une quelconque des revendications 1 à 16, dans lequel dans l'étape (iii), lesdits un ou plusieurs réactants sont mis en oeuvre dans un tampon semi volatil, et comprenant en outre l'étape, après la fin de la réaction, d'élimination du tampon semi volatil.

20. Procédé selon l'une quelconque des revendications précédentes, dans lequel les enzymes sont attachées à la sonde en tant que protéines de fusion, typiquement par une étiquette.

21. Procédé selon l'une quelconque des revendications précédentes, dans lequel ledit composé de test est ajouté avant, après ou avec le ou les réactants pour déterminer son effet sur l'activité enzymatique.

22. Procédé selon l'une quelconque des revendications précédentes, dans lequel le ou les réactants et le composé de test éventuel sont introduits au niveau de l'enzyme immobilisée en tant que gouttelette, telle qu'une gouttelette ayant un volume de moins de 1 microlitre.
